# EUROPEAN PATENT APPLICATION

(11) **EP 1 329 218 A1**
(43) Date of publication of application: **23.07.2003**
(21) Application number: 01978864.5
(22) Date of filing: 24.10.2001
(51) Int. Cl.: A61K 31/41, A61K 31/4178, A61K 31/4184, A61K 31/4245, A61K 31/433, A61K 45/00, A61P 43/00, A61P 9/12, C07D 401/10

(54) **PREVENTIVES/REMEDIES FOR PORTAL HYPERTENSION**

(30) Priority: 25.10.2000 JP 2000326131
(71) Applicant: Takeda Chemical Industries, Ltd., Osaka-shi, Osaka 541-0045 (JP)
(72) Inventor: WATANABE, Toshifumi, Kawachinagano-shi, Osaka 586-0092 (JP); KUSUMOTO, Keiji, Mishima-gun, Osaka 618-0011 (JP); OJIMA, Mami, Amagasaki-shi, Hyogo 661-0002 (JP)
(74) Representative: Teipel, Stephan, Dr.
(86) International application number: JP0109311
(87) International publication number: WO02034263

(57) **Abstract**

An agent for preventing or treating portal hypertension comprising a compound represented by the general formula (I): 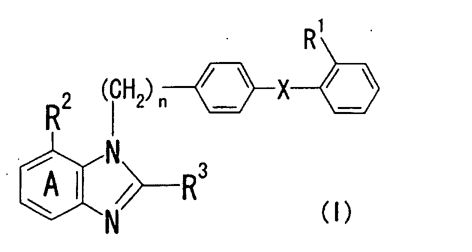 wherein R¹ represents a group capable of forming an anion, etc.; X represents a bond or a spacer; n is an integer of 1 or 2; ring A is benzene ring which may be further substituted; R² represents a group capable of forming an anion, etc.; and R³ represents a hydrocarbon group which may bonded via a hetero atom, and may be substituted, a salt thereof or a prodrug thereof, is provided. This agent has sufficiently excellent properties as medicine, since it has excellent prophylactic and therapeutic effects on portal hypertension without any side effect, etc.

## Description

### TECHNICAL FIELD

The present invention relates to an agent for preventing or treating portal hypertension comprising as an effective component a benzimidazole derivative having angiotensin II antagonistic activity (All antagonistic activity), or a salt thereof, or a prodrug thereof; a sustained release agent for preventing or treating portal hypertension comprising a compound having All antagonistic activity, or a salt thereof, or a prodrug thereof; and the like.

### PRIOR ART

Portal hypertension means a state in which portal vein pressure has risen due to stenosis or occlusion of the portal vein, sinusoid, lever vein and the like. Portal hypertension causes pooling of abdominal dropsy, esophageal varices, gastric varices and the like, and especially, hemorrhage per rhexis of varices has a strong possibility of being a fatal wound. Therefore, early prevention has been required. As a vasoconstrictor involved in a rise of portal vein pressure, All is exemplified (Gastroenterology 2000; 118: 1261-1265). That is, portal vein pressure of an isolated liver preparation is risen by administration of exogenous All (J. Pharmacol. Exp. Thr. 1988; 244, 283-289). Furthermore, under hepatopathy, a sinusoid cell, which is one of cells that determines the resistance of portal vein, is contracted by stimulation with AT1 (Gastroenterology 2000; 118: 1149-1156).

Benzimidazole derivatives having All antagonistic activity are known to be an agent for treating cardiovascular diseases such as hypertension, cardiac diseases (cardiac hypertrophy, cardiac failure, cardiac infarction and the like), cerebral stroke, nephritis, and the like (JP 4-364171 A and the like), which shows a long-lasting anti-hypertensive activity by preventing AII, which induces a strong vascular constriction, from reacting an All receptor.

On the other hand, among compounds having All antagonistic activity, there is a report, which suggests the application of Losartan to portal hypertension [Hepatology 1999; 29: 334-339]. However, there has been no report, which suggests that a benzimidazole derivative having All antagonistic activity shows effects for preventing or treating portal hypertension.

Furthermore, a sustained release preparation comprising a benzimidazole derivative having All antagonistic activity is disclosed in WO99/44590 and JP 11-351798 A. However, there is no suggestion that the sustained release preparation shows effects for preventing or treating portal hypertension.

Therefore, it is desired to develop an agent for preventing or treating portal hypertension, which has sufficiently excellent properties as medicine, for example, superior effects for preventing or treating portal hypertension, and shows no side effects, and the like.

### SUMMARY OF THE INVENTION

Under these circumstances, the present inventors have conducted intensive studies on drugs having an effect for preventing or treating portal hypertension. As a result, they have found that a compound having angiotensin II (AII) antagonistic activity, which is represented by a specific structural formula, is extremely effective, and that when a sustained release preparation containing the compound having All antagonistic activity is used, unexpectedly superior prophylactic or therapeutic effect can. be obtained, and the like. They have conducted further studies on that finding, which resulted in the completion of the present invention.

Namely, the present invention relates to:
(1) An agent for preventing or treating portal hypertension which comprises a compound represented by the formula (I): 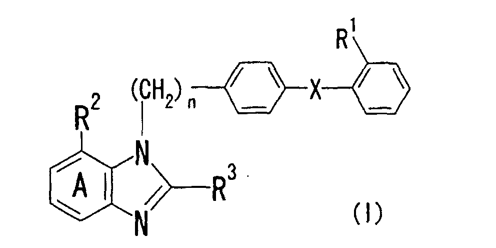 wherein R¹ represents a group capable of forming an anion or a group capable of converting to said group, X represents that the phenylene group and the phenyl group are linked directly or via a spacer having a chain of two or less of atoms, n represents 1 or 2, ring A represents a benzene ring which may be further substituted, R² represents a group capable of forming an anion or a group capable of converting to said group, R³ represents a hydrocarbon group which may link via a heteroatom and may be substituted, or a salt thereof, or a prodrug thereof;
(2) An agent for preventing or treating portal hypertension comprising 2-ethoxy-1-[[2'-(1H-tetrazol-5-yl)biphenyl-4-yl]methyl]benzimidazole-7-carboxylic acid, or a salt thereof, or a prodrug thereof;
(3) An agent for preventing or treating portal hypertension comprising 2-ethoxy-1-[[2'-(2,5-dihydro-5-oxo-1,2,4-oxadiazol-3-yl)biphenyl-4-yl]methyl]benzimidazole-7-carboxylic acid, or a salt thereof, or a prodrug thereof;
(4) An agent for preventing or treating portal hypertension comprising 1-(cyclohexyloxycarbonyloxy)ethyl 2-ethoxy-1-[[2'-(1H-tetrazol-5-yl)biphenyl-4-yl]methyl]benzimidazole-7-carboxylate or a salt thereof;
(5) A sustained release agent for preventing or treating portal hypertension comprising a compound having All antagonistic activity, or a salt thereof, or a prodrug thereof;
(6) The agent according to the above (5), wherein the compound having All antagonistic activity is a compound represented by the formula (I): 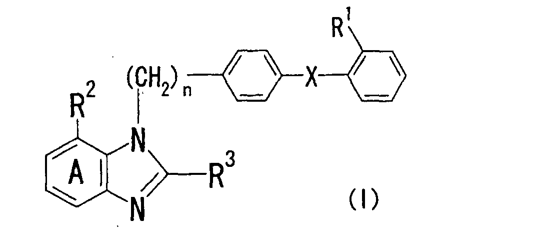 wherein R¹ represents a group capable of forming an anion or a group capable of converting to said group, X represents that the phenylene group and the phenyl group are linked directly or via a spacer having a chain of two or less of atoms, n represents 1 or 2, ring A represents a benzene ring which may be further substituted, R² represents a group capable of forming an anion or a group capable of converting to said group, R³ represents a hydrocarbon group which may link via a heteroatom and may be substituted;
(7) The agent according to the above (5), wherein the compound having All antagonistic activity is 2-ethoxy-1-[[2'-(1H-tetrazol-5-yl)biphenyl-4-yl]methyl]benzimidazole-7-carboxylic acid;
(8) The agent according to the above (5), wherein the compound having All antagonistic activity is 2-ethoxy-1-[(2'-(2,5-dihydro-5-oxo-1,2,4-oxadiazol-3-yl)biphenyl-4-yl]methyl]benzimidazole-7-carboxylic acid;
(9) The agent according to the above (5), wherein the compound having All antagonistic activity is 1-(cyclohexyloxycarbonyloxy)ethyl 2-ethoxy-1-[[2'-(1H-tetrazol-5-yl)biphenyl-4-yl]methyl]benzimidazole-7-carboxylate;
(10) The agent according to the above (5), wherein the compound having All antagonistic activity or a salt thereof is Losartan, Potassium Losartan, Eprosartan, Candesartan cilexetil, Candesartan, Valsartan, Telmisartan, Irbesartan, Olmesartan or Tasosartan;
(11) A method for preventing or treating portal hypertension which comprises administering an effective amount of a sustained release agent for preventing or treating portal hypertension comprising a compound having All antagonistic activity, or a salt thereof, or a prodrug thereof to a mammal;
(12) Use of a compound having All antagonistic activity, or a salt thereof, or a prodrug thereof for manufacturing a sustained release agent for preventing or treating portal hypertension;
(13) A method for suppressing hepatic fibrosis which comprises administering an effective amount of a sustained release agent comprising a compound having All antagonistic activity, or a salt thereof, or a prodrug thereof to a mammal;
(14) A method for preventing or treating portal hypertension which comprises administering an agent for preventing or treating portal hypertension comprising a compound represented by the formula (I): 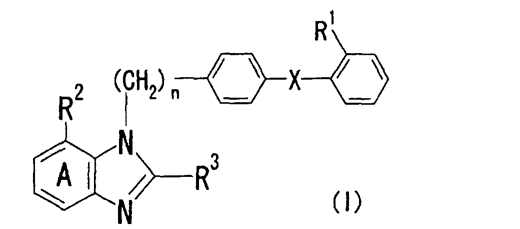 wherein R¹ represents a group capable of forming an anion or a group capable of converting to said group, X represents that the phenylene group and the phenyl group are linked directly or via a spacer having a chain of two or less of atoms, n represents 1 or 2, ring A represents a benzene ring which may be further substituted, R² represents a group capable of forming an anion or a group capable of converting to said group, R³ represents a hydrocarbon group which may link via a heteroatom and may be substituted, or a salt thereof, or a prodrug thereof, to a mammal;
(15) Use of a compound represented by the formula (I): 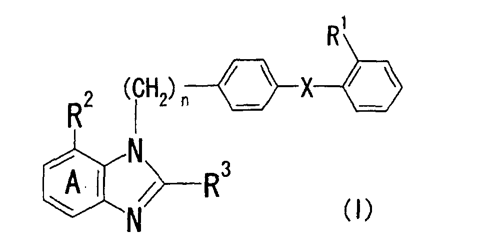
wherein R¹ represents a group capable of forming an anion or a group capable of converting to said group, X represents that the phenylene group and the phenyl group are linked directly or via a spacer having a chain of two or less of atoms, n represents 1 or 2, ring A represents a benzene ring which may be further substituted, R² represents a group capable of forming an anion or a group capable of converting to said group, R³ represents a hydrocarbon group which may link via a heteroatom and may be substituted, or a salt thereof, or a prodrug thereof, for manufacturing an agent for preventing or treating portal hypertension. ; and the like.

### EMBODIMENTS FOR CARRYING OUT THE INVENTION

In the present invention, a benzimidazole derivative represented by the formula (I): 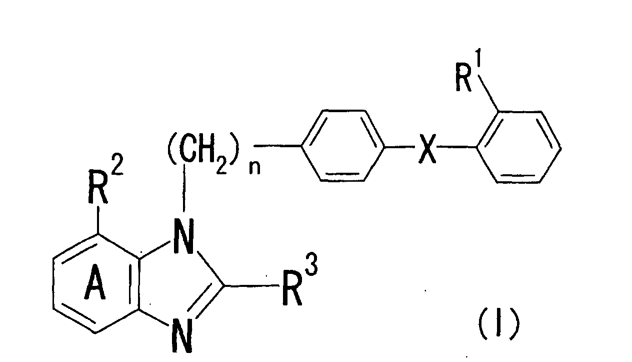 wherein R¹ represents a group capable of forming an anion or a group capable of converting to said group, X represents that the phenylene group and the phenyl group are linked directly or via a spacer having a chain of two or less of atoms, n represents 1 or 2, ring A represents a benzene ring which may be further substituted, R² represents a group capable of forming an anion or a group capable of converting to said group, R³ represents a hydrocarbon group which may link via a heteroatom and may be substituted (preferably, a hydrocarbon group which may be substituted and may b link via oxygen atom), or a salt thereof, or a prodrug thereof, and the like, is preferably used as an effective component. In the above-mentioned formula (I), examples of the group capable of forming an anion (a group having a hydrogen atom which may be liberated as a proton) of R¹ include (1) carboxyl group, (2) tetrazolyl group, (3) trifluoromethanesulfonic acid amide group (-NHSO₂CF₃), (4) phosphoric acid group, (5) sulfonic acid group, (6) an optionally substituted 5- to 7-membered (preferably 5- to 6-membered) monocyclic heterocyclic residue containing one or two or more of N, S and O, and the like.

Examples of the above-mentioned "optionally substituted 5- to 7-membered (preferably 5- to 6-membered) monocyclic heterocyclic residue containing one or two or more of N, S and O" include 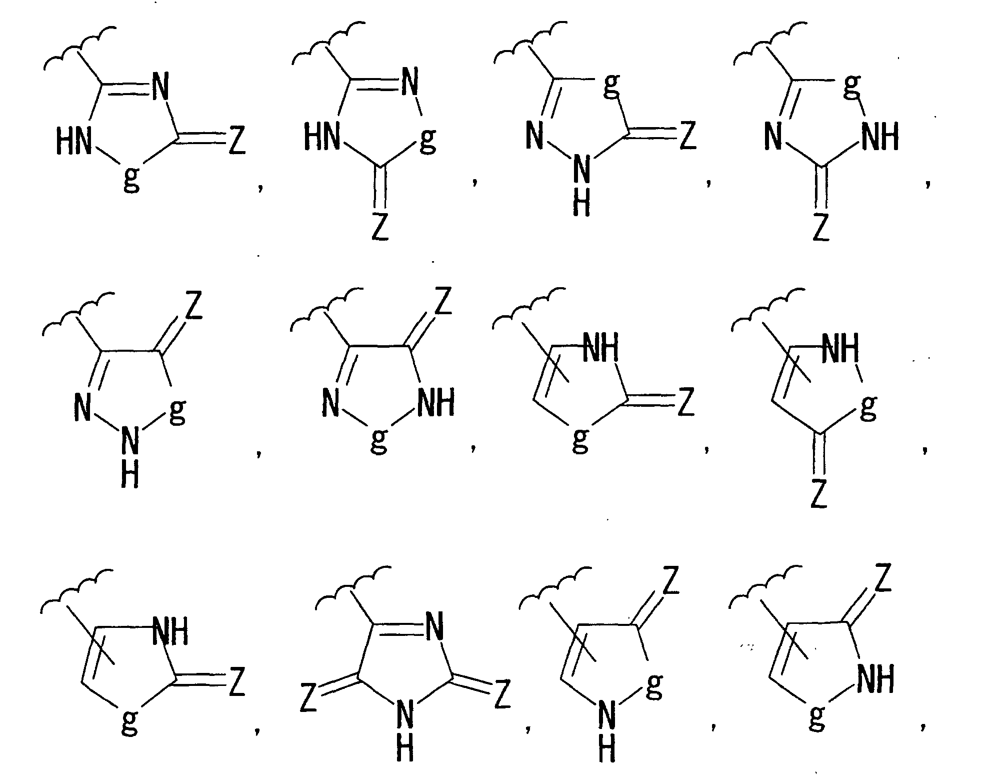 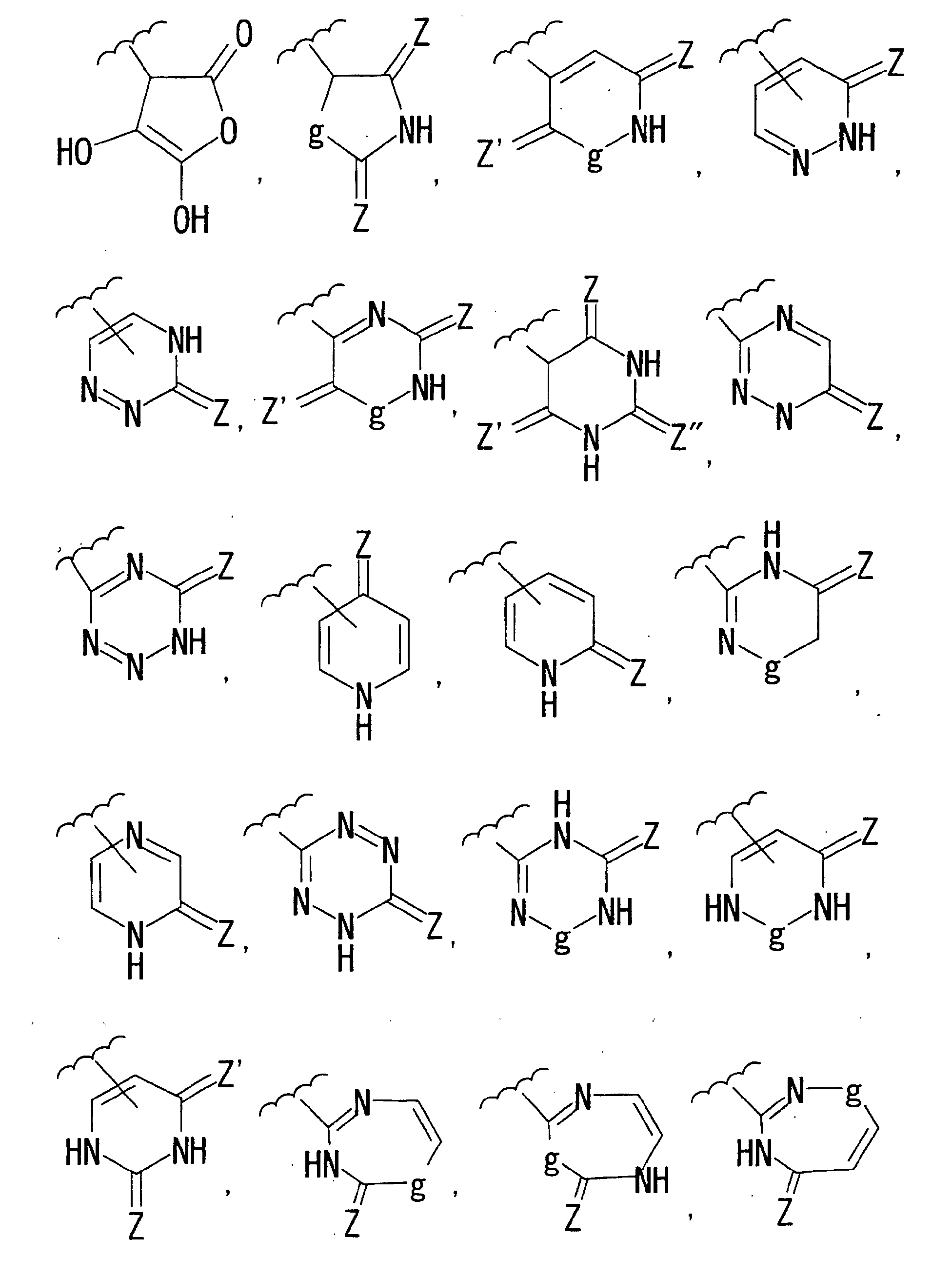 and the like. Furthermore, when g in the above-mentioned formula represents -NH- and the like, the linkage between the heterocyclic residue represented by R¹ and the phenyl group to which the heterocyclic residue is linked, may be not only the above-mentioned carbon-carbon bond, but also a bond via one of nitrogen atoms whenever plural nitrogen atoms are present. Specifically, when R¹ is represented by the formula: 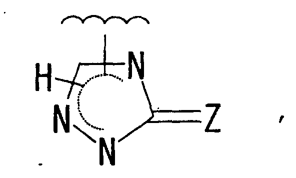 it may be a group represented by 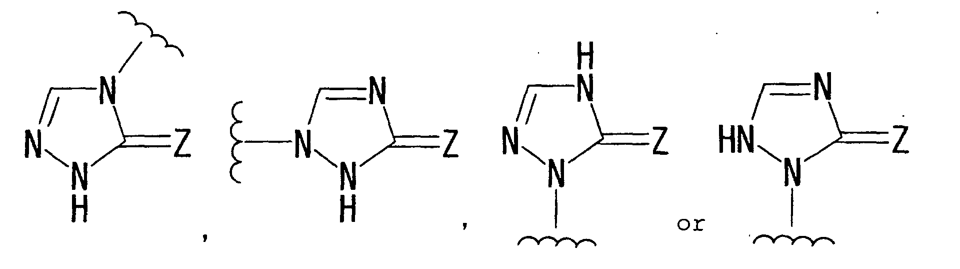 respectively. Other examples of the link via a nitrogen atom include 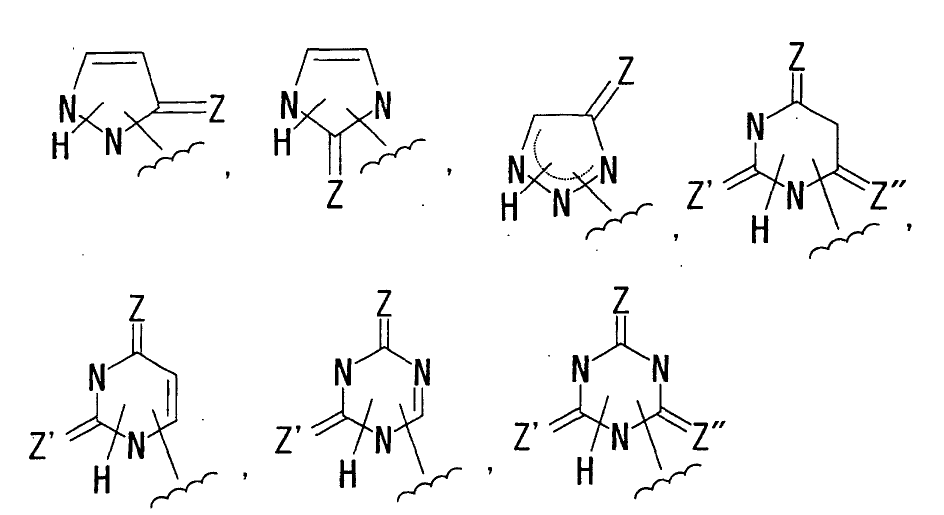 and the like.

In the above-mentioned formulas, g represents -CH₂-,-NH-, -O- or -S(O)ₘ-; >=Z, >=Z' and >=Z'' each represents carbonyl group, thiocarbonyl group or optionally oxidized sulfur atom (e.g., S, S(O), S(O)₂ and the like) (preferably carbonyl or thiocarbonyl group, more preferably carbonyl group), respectively, and m represents 0, 1 or 2.

As the heterocyclic residue represented by R¹, a group obtained by eliminating one hydrogen atom from a ring that has -NH- or -OH group as a proton donor and carbonyl group, thiocarbonyl group or sulfinyl group and the like as a proton acceptor, simultaneously, such as oxadiazolone ring, oxadiathiazolone ring or thiadiazolone group, and the like, are preferred. Furthermore, the heterocyclic residue represented by R¹ may form a fused ring group by fusion with a cyclic substituent, while a 5- or 6-membered ring residue is preferred, and a 5-membered ring residue is more preferred as the heterocyclic residue represented by R¹.

As the heterocyclic residue represented by R¹, a group represented by the formula: 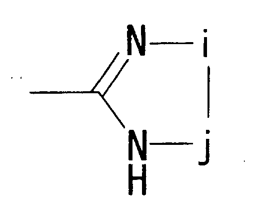 wherein i represents -O- or -S-, j represents >=O,>=S or >=S(O)ₘ, m is as defined above (among these, 2,5-dihydro-5-oxo-1,2,4-oxadiazol-3-yl, 2,5-dihydro-5-thioxo-1,2,4-oxadiazol-3-yl, 2,5-dihydro-5-oxo-1,2,4-thiadiazol-3-yl, especially 2,5-dihydro-5-oxo-1,2,4-oxadiazol-3-yl) and the like are preferred.

Furthermore, as represented below, the above-mentioned heterocyclic residue (R¹) has tautomers. For example, when Z=O and g=O in the following formula: 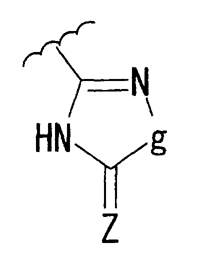 three tautomers a', b' and c', such as 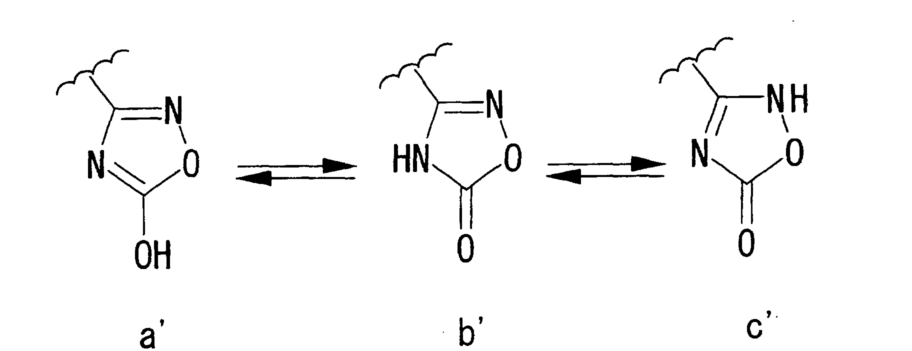 are exist, and the heterocyclic residue represented by the formula: 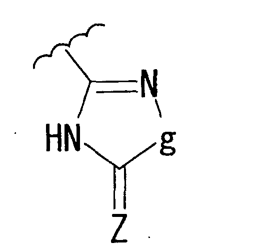 includes all of the above-mentioned a', b' and c'.

The group capable of forming an anion of R¹ may be protected with an optionally substituted lower(C₁₋₄)alkyl group or an acyl group (e.g., lower(C₂₋₅)alkanoyl, benzoyl and the like) and the like, at any possible position(s).

Examples of the optionally substituted lower(C₁₋₄)alkyl group include (1) a lower(C₁₋₄)alkyl group which may be substituted with 1 to 3 phenyl groups which may have halogen atom, nitro, lower(C₁₋₄)alkyl, lower(C₁₋₄)alkoxy and the like (e.g., methyl, triphenylmethyl, p-methoxybenzyl, p-nitrobenzyl and the like), (2) a lower(C₁₋₄)alkoxy-lower(C₁₋₄)alkyl group (e.g., methoxymethyl, ethoxymethyl and the like), (3) formula: -CH(R⁴)-OCOR⁵ [wherein R⁴ represents (a) hydrogen, (b) a straight or branched lower alkyl group having 1-6 carbon atoms (e.g., methyl, ethyl, n-propyl, isopropyl, n-butyl, isobutyl, t-butyl, n-pentyl, isopentyl, neopentyl and the like), (c) a straight or branched lower alkenyl group having 2-6 carbon atoms or (d) a cycloalkyl group having 3-8 carbon atoms (e.g., cyclopentyl, cyclohexyl, cycloheptyl and the like), R⁵ represents (a) a straight or branched lower alkyl group having 1-6 carbon atoms (e.g., methyl, ethyl, n-propyl, isopropyl, n-butyl, isobutyl, sec-butyl, t-butyl, n-pentyl, isopentyl, neopentyl and the like), (b) a straight or branched lower alkenyl group having 2-6 carbon atoms, (c) a lower alkyl group having 1 to 3 carbon atom(s) substituted with a cycloalkyl group having 3-8 carbon atoms (e.g., cyclopentyl, cyclohexyl, cycloheptyl and the like) or an optionally substituted aryl group (e.g., phenyl or naphthyl group and the like, each of which may have halogen atom, nitro, lower(C₁₋₄)alkyl, lower(C₁₋₄)alkoxy and the like) (e.g., benzyl, p-chlorobenzyl, phenethyl, cyclopentylmethyl, cyclohexylmethyl and the like), (d) a lower alkenyl group having 2 to 3 carbon atoms substituted by cycloalkyl having 3-8 carbon atoms or an optionally substituted aryl group (e.g., phenyl or naphthyl group and the like, each of which may have halogen atom, nitro, lower(C₁₋₆)alkyl, lower(C₁₋ ₄)alkoxy and the like) (e.g., a group having alkenyl portion(s) such as vinyl, propenyl, allyl, isopropenyl and the like, such as cynnamyl, etc., and the like), (e) an optionally substituted aryl group (e.g., phenyl or naphthyl group and the like, each of which may have halogen atom, nitro, lower(C₁₋₄)alkyl, lower(C₁₋₄)alkoxy and the like, such as phenyl, p-tolyl, naphthyl and the like), (f) a straight or branched lower alkoxy group having 1-6 carbon atoms (e.g., methoxy, ethoxy, n-propoxy, isopropoxy, n-butoxy, isobutoxy, sec-butoxy, t-butoxy, n-pentyloxy, isopentyloxy, neopentyloxy and the like), (g) a straight or branched lower alkenyloxy group having 2 to 8 carbon atoms (e.g., allyloxy, isobutenyloxy and the like), (h) a cycloalkyloxy group having 3-8 carbon atoms (e.g., cyclopentyloxy, cyclohexyloxy, cycloheptyloxy and the like), (i) a lower alkoxy group having 1 to 3 carbon atoms substituted with cycloalkyl having 3-8 carbon atoms (e.g., cyclopentyl, cyclohexyl, cycloheptyl and the like) or an optionally substituted aryl group (e.g., phenyl or naphthyl group and the like, each of which may have halogen atom, nitro, lower(C₁₋₄)alkyl, lower(C₁₋₄)alkoxy and the like) (e.g., a group having alkoxy portion(s) such as methoxy, ethoxy, n-propoxy, isopropoxy and the like, such as benzyloxy, phenethyloxy, cyclopentylmethoxy, cyclohexylmethoxy and the like), (j) a lower alkenyloxy group having 2 to 3 carbon atoms substituted with cycloalkyl having 3-8 carbon atoms (e.g., cyclopentyl, cyclohexyl, cycloheptyl and the like) or an optionally substituted aryl group (e.g., phenyl or naphthyl group and the like, each of which may have halogen atom, nitro, lower(C₁₋₄)alkyl, lower(C₁₋₄)alkoxy and the like) (e.g., a group having alkenyloxy portion(s) such as vinyloxy, propenyloxy, allyloxy, isopropenyloxy and the like, such as cynnamyloxy and the like) or (k) optionally substituted aryloxy group (e.g., phenoxy or naphthoxy group and the like, each of which may have halogen atom, nitro, lower(C₁₋₄)alkyl, lower(C₁₋₄)alkoxy and the like, for example, phenoxy, p-nitrophenoxy, naphthoxy and the like)] and the like.

Furthermore, the group capable of forming an anion of R¹ may have substituent(s) such as an optionally substituted lower(C₁₋₄)alkyl group (which includes the groups similar to the "optionally substituted lower(C₁₋ ₄)alkyl group" exemplified as a protective group for the group capable of forming an anion of the above-mentioned R¹), halogen atom, nitro, cyano, lower(C₁₋₄)alkoxy, amino optionally substituted with 1 or 2 of lower(C₁₋₄)alkyls and the like, at any possible position(s), in addition to the protective group such as the above-mentioned optionally substituted lower(C₁₋₄)alkyl group or acyl group (e.g., lower(C₂₋₅)alkanoyl, benzoyl and the like).

In the above-mentioned formula, the group capable of converting to the group capable of forming an anion of R¹ (a group having hydrogen atom which may be liberated as a proton) may be a group capable of converting to the group capable of forming an anion by an in vivo reaction and the like, such as a reaction under biological, i.e., physiological conditions (for example, oxidation, reduction or hydrolysis and the like with an in vivo enzyme and the like (so-called prodrug), or may be a group capable of converting to the group capable of forming an anion represented by R¹ by a chemical reaction (so-called synthetic intermediate) such as (1) carboxyl group, (2) tetrazolyl group, (3) trifluoromethanesulfonic acid amide group (-NHSO₂CF₃), (4) phosphoric acid group, (5) sulfonic acid group, (6) optionally substituted 5- to 7-membered (preferably 5- to 6-membered) monocyclic heterocyclic residue containing one or two or more of N, S and O, each of which has been protected with cyano, N-hydroxycarbamimidoyl group (-C(=N-OH)-NH₂) or an optionally substituted lower(C₁₋₄)alkyl group or acyl group.

As R¹, carboxyl optionally protected with optionally substituted lower(C₁₋₄)alkyl (e.g., methyl, triphenylmethyl, methoxymethyl, ethoxymethyl, p-methoxybenzyl, p-nitrobenzyl and the like) or acyl group (e.g., lower(C₂₋₅)alkanoyl, benzoyl and the like), tetrazolyl or 2,5-dihydro-5-oxo-1,2,4-oxadiazol-3-yl (preferably tetrazolyl), or cyano, N-hydroxycarbamimidoyl (preferably cyano), are preferred, and in particular, cyano is preferably used.

In the above-mentioned formula, X represents that the adjacent phenylene group and phenyl group are linked directly or via a spacer having a chain of two or less atom(s) (preferably linked directly), and the spacer having a chain of two or less of atom(s) may be any chain so long as a divalent chain having the number of the atom constituting the straight chain portion of 1 or 2. The chain may also have side chain(s). Specifically, it includes lower(C₁₋₄)alkylene having 1 or 2 atom(s) constituting the straight chain portion, such as -CO-, -O-, -S-, -NH-, -CO-NH-, -O-CH₂-, -S-CH₂-, -CH=CH- and the like,

In the above-mentioned formula, n represents an integer of 1 or 2 (preferably 1).

In the above-mentioned formula, ring A represents a benzene ring which may be further substituted in addition to the substituent R², and examples of said substituent include (1) halogen (e.g., F, Cl, Br and the like), (2) cyano, (3) nitro, (4) optionally substituted lower(C₁₋ ₄)alkyl, (5) lower(C₁₋₄)alkoxy, (6) optionally substituted amino group (e.g., amino, N-lower(C₁₋₄)alkylamino (e.g., methylamino and the like), N,N-di-lower(C₁₋₄)alkylamino (e.g., dimethylamino and the like), N-arylamino (e.g., phenylamino and the like), alicyclic amino (e.g., morpholino, piperidino, piperazino, N-phenylpiperazino and the like) and the like), (7) a group represented by the formula: -CO-D' [wherein D' represents hydroxy group or lower(C₁₋₄)alkoxy in which the alkyl portion is optionally substituted with hydroxy group, lower(C₁₋₄)alkoxy, lower(C₂₋ ₆)alkanoyloxy (e.g., acetoxy, pivaloyloxy and the like), lower (C₁₋₆)alkoxycarbonyloxy (e.g., methoxycarbonyloxy, ethoxycarbonyloxy and the like) or lower(C₃₋ ₆)cycloalkoxycarbonyloxy (e.g., cyclohexyloxycarbonyloxy and the like)], or (8) optionally substituted lower(C₁₋ ₄)alkyl (including the groups similar to the "optionally substituted lower(C₁₋₄)alkyl group" exemplfied as protective groups for the group capable of froming an anion of the above-mentioned R¹) or tetrazolyl which may protected with an acyl (e.g., lower(C₂₋₅)alkanoyl, benzoyl and the like), trifluoromethanesulfonic acid amide group, phosphoric acid group or sulfonic acid group and the like.

One or two of these substituent(s) may be present at any possible position(s) of the benzene ring, and examples of the substituent which is further possessed by ring A in addition to the substituent R² include optionally substituted lower(C₁₋₄)alkyl (e.g., lower(C₁₋₄)alkyl optionally substituted with hydroxy group, carboxyl group, halogen, etc., and the like), halogen and the like, and more preferably, ring A does not have any substituent except for the substituent R².

In the above-mentioned formula, examples of a group capable of forming an anion of R² (a group having a hydrogen atom which may be liberated as a proton) include (1) carboxyl group which may be esterified or amidated, (2) tetrazolyl group, (3) trifluoromethanesulfonic acid amide group (-NHSO₂CF₃), (4) phosphoric acid group, (5) sulfonic acid group and the like. These groups may be protected with an optionally substituted lower alkyl group (including the similar groups to the "optionally substituted lower(C₁₋ ₄)alkyl group" exemplified as protective groups for the group capable of forming an anion of the above-mentioned R¹) or an acyl group (e.g., lower(C₂₋₅)alkanoyl, benzoyl and the like), and any of them can be used in so far as the group can be converted to a group capable of forming an anion under biological, i.e., physiological conditions (for example, oxidation, reduction or hydrolysis and the like with an in vivo enzyme and the like), or can be chemically converted to a group capable of forming an anion.

Examples of the carboxyl which may be esterified or amidated of R² include a group represented by the formula: -CO-D [wherein D represents (1) hydroxy group, (2) optionally substituted amino (for example, amino, N-lower(C₁₋₄)alkylamino, N,N-dilower(C₁₋₄)alkylamino and the like) or (3) optionally substituted alkoxy {e.g., (i) lower(C₁₋₆)alkoxy group in which the alkyl portion is optionally substituted with hydroxy group, optionally substituted amino (e.g., amino, N-lower (C₁₋₄) alkylamino, N,N-dilower(C₁₋₄)alkylamino, piperidino, morpholino and the like), halogen, lower(C₁₋₆)alkoxy, lower(C₁₋₆)alkylthio, lower(C₃₋₈)cycloalkoxy or optionally substituted dioxolenyl (e.g., 5-methyl-2-oxo-1,3-dioxolen-4-yl and the like), or (ii) a group of the formula: -O-CH(R⁶)-OCOR⁷ [wherein R⁶ represents (a) hydrogen, (b) a straight or branched lower alkyl group having 1-6 carbon atom(s) (e.g., methyl, ethyl, n-propyl, isopropyl, n-butyl, isobutyl, t-butyl, n-pentyl, isopentyl, neopentyl and the like), (c) a straight or branched lower alkenyl group having 2-6 carbon atoms or (d) a cycloalkyl group having 3-8 carbon atoms (e.g., cyclopentyl, cyclohexyl, cycloheptyl and the like), R⁷ represents (a) a straight or branched lower alkyl group having 1-6 carbon atom(s) (e.g., methyl, ethyl, n-propyl, isopropyl, n-butyl, isobutyl, sec-butyl, t-butyl, n-pentyl, isopentyl, neopentyl and the like), (b) a straight or branched lower alkenyl group having 2-6 carbon atoms, (c) a lower alkyl group having 1 to 3 carbon atom(s) substituted with a cycloalkyl group having 3-8 carbon atoms (e.g., cyclopentyl, cyclohexyl, cycloheptyl and the like) or optionally substituted aryl group (e.g., phenyl or naphthyl group and the like, each of which may have halogen atom, nitro, lower(C₁₋₄)alkyl, lower(C₁₋₄)alkoxy and the like) (e.g., benzyl, p-chlorobenzyl, phenethyl, cyclopentylmethyl, cyclohexylmethyl and the like), (d) a lower alkenyl group having 2 to 3 carbon atoms substituted with cycloalkyl having 3-8 carbon atoms or an optionally substituted aryl group (e.g., phenyl or naphtyl group and the like, each of which may have halogen atom, nitro, lower(C₁₋₄)alkyl, lower(C₁₋₄)alkoxy and the like) (e.g., a group having alkenyl portion(s) such as vinyl, propenyl, allyl, isopropenyl and the like, such as cinnamyl and the like), (e) an optionally substituted aryl group (e.g., phenyl or naphthyl group and the like, each of which may have halogen atom, nitro, lower(C₁₋₄)alkyl, lower(C₁₋₄)alkoxy and the like, such as phenyl, p-tolyl, naphthyl and the like), (f) a straight or branched lower alkoxy group having 1-6 carbon atoms (e.g., methoxy, ethoxy, n-propoxy, isopropoxy, n-butoxy, isobutoxy, sec-butoxy, t-butoxy, n-pentyloxy, isopentyloxy, neopentyloxy and the like), (g) a straight or branched lower alkenyloxy group having 2 to 8 carbon atoms (e.g., allyloxy, isobutenyloxy and the like), (h) a cycloalkyloxy group having 3-8 carbon atoms (e.g., cyclopentyloxy, cyclohexyloxy, cycloheptyloxy and the like), (i) a lower alkoxy group having 1 to 3 carbon atoms substituted with cycloalkyl having 3-8 carbon atoms (e.g., cyclopentyl, cyclohexyl, cycloheptyl and the like) or an optionally substituted aryl group (e.g., phenyl or naphthyl group and the like, each of which may have halogen atom, nitro, lower(C₁₋₄)alkyl, lower(C₁₋₄)alkoxy .and the like) (e.g., a group having alkoxy portion(s) such as methoxy, ethoxy, n-propoxy, isopropoxy and the like, such as benzyloxy, phenethyloxy, cyclopentylmethoxy, cyclohexylmethoxy, etc., and the like), (j) a lower alkenyloxy group having 2 to 3 carbon atoms substituted with cycloalkyl having 3-8 carbon atoms (e.g., cyclopentyl, cyclohexyl, cycloheptyl and the like) or an optionally substituted aryl group (e.g., phenyl or naphthyl group and the like, each of which may have halogen atom, nitro, lower(C₁₋₄)alkyl, lower(C₁₋₄)alkoxy and the like) (e.g., a group having alkenyloxy portion(s) such as vinyloxy, propenyloxy, allyloxy, isopropenyloxy and the like, such as cinnamyloxy and the like, and the like) or (k) optionally substituted aryloxy group (e.g., phenoxy or naphthoxy group and the like, each of which may have halogen atom, nitro, lower(C₁₋₄)alkyl, lower(C₁₋₄)alkoxy and the like, for example, phenoxy, p-nitro phenoxy, naphthoxy and the like)] and the like}] and the like.

As R², carboxyl which may be esterified is preferred, and the specific examples thereof include -COOH and a salt thereof, -COOMe, -COOEt, -COOtBu, -COOPr, pivaloyloxymethoxycarbonyl, 1-(cyclohexyloxycarbonyloxy)ethoxycarbonyl, 5-methyl-2-oxo-1,3-dioxolen-4-ylmethoxycarbonyl, acetoxymethoxycarbonyl, propionyloxymethoxycarbonyl, n-butyryloxymethoxycarbonyl, isobutyryloxymethoxycarbonyl, 1-(ethoxycarbonyloxy)ethoxycarbonyl, 1-(acetoxy)ethoxycarbonyl, 1-(isobutyryloxy)ethoxycarbonyl, cyclohexylcarbonyloxymethoxycarbonyl, benzoyloxymethoxycarbonyl, cinnamyloxycarbonyl, cyclopentylcarbonyloxymethoxycarbonyl and the like, and any of them can be used in so far as the group can be converted to a group capable of forming an anion (e.g., COO⁻, its derivative, etc.) or a group capable of convert thereto under biological, i.e., physiological conditions (for example, oxidation, reduction or hydrolysis and the like with an in vivo enzyme and the like), or chemically. The group may be carboxyl group or a prodrug form thereof.

As the above-mentioned R², a group represented by the formula: -CO-D [wherein D represents (1) hydroxy group or (2) lower(C₁₋₄)alkoxy in which the alkyl portion is optionally substituted with hydroxy group, amino, halogen, lower(C₂₋₆)alkanoyloxy (e.g., acetoxy, pivaloyloxy and the like), lower (C₃₋₈)cycloalkanoyloxy, lower (C₁₋ ₆)alkoxycarbonyloxy (e.g., methoxycarbonyloxy, ethoxycarbonyloxy and the like), lower(C₃₋ ₈)cycloalkoxycarbonyloxy (e.g., cyclohexyloxycarbonyloxy and the like), lower (C₁₋₄)alkoxy or lower(C₃₋₈)cycloalkoxy] is preferred. Among these, carboxyl esterified with lower(C₁₋₄)alkyl (preferably, methyl or ethyl) is preferred.

In the above-mentioned formula, examples of the "hydrocarbon residue" in the "hydrocarbon residue which may link via a heteroatom and may be substituted" represented by R³ include (1) an alkyl group, (2) an alkenyl group, (3) an alkynyl group, (4) a cycloalkyl group, (5) an aryl group, (6) an aralkyl group and the like. Among these, an alkyl group, an alkenyl group and a cycloalkyl group are preferred.

The alkyl group of the above-mentioned (1) may be any of straight or branched lower alkyl groups having 1 to 8 carbon atoms or so and examples thereof include methyl, ethyl, propyl, isopropyl, butyl, isobutyl, sec-butyl, t-butyl, pentyl, i-pentyl, hexyl, heptyl, octyl and the like.

The alkenyl group of the above-mentioned (2) may be any of straight or branched lower alkenyl groups having 2 to 8 carbon atoms or so and examples thereof include vinyl, propenyl, 2-butenyl, 3-butenyl, isobutenyl, 2-octenyl and the like.

The alkynyl group of the above-mentioned (3) may be any of straight or branched lower alkynyl groups having 2 to 8 carbon atoms or so and examples thereof include ethynyl, 2-propynyl, 2-butynyl, 2-pentynyl, 2-octynyl and the like.

Examples of the cycloalkyl group of the above-mentioned (4) include lower cycloalkyl having about 3 to 6 carbon atoms or so, such as cyclopropyl, cyclobutyl, cyclopentyl, cyclohexyl and the like.

Each of the above-mentioned alkyl group, alkenyl group, alkynyl group and cycloalkyl group may be substituted with hydroxy group, an optionally substituted amino group (e.g., amino, N-lower(C₁₋₄)alkylamino, N,N-dilower(C₁₋₄)alkylamino and the like), halogen, a lower(C₁₋₄)alkoxy group, a lower(C₁₋₄)alkylthio group and the like.

Examples of the lower cycloalkyl of the above-mentioned (5) include phenyl-lower(C₁₋₄)alkyl and the like such as benzyl, phenethyl and the like, and examples of the aryl group of the above-mentioned (6) include phenyl and the like.

Each of the above-mentioned aralkyl group or aryl group may be substituted with, for example, halogen (e.g., F, Cl, Br and the like), nitro, optionally substituted amino group (e.g., amino, N-lower(C₁₋₄)alkylamino, N,N-dilower(C₁₋₄)alkylamino and the like), lower(C₁₋₄)alkoxy (e.g., methoxy, ethoxy and the like), lower(C₁₋₄)alkylthio (e.g., methylthio, ethylthio and the like), lower(C₁₋₄)alkyl (e.g., methyl, ethyl and the like) and the like, at any possible position(s) on the benzene ring.

Among the above-mentioned groups, as the "hydrocarbon residue" in the "hydrocarbon residue which may link via a heteroatom and may be substituted" represented by R³, an optionally substituted alkyl or an alkenyl group (e.g., lower(C₁₋₅)alkyl or lower(C₂₋₅)alkenyl group and the like, each of which is optionally substituted with hydroxy group, amino group, halogen or lower(C₁₋₄)alkoxy group) are preferred. Among these, lower(C₁₋₅)alkyl (more preferably ethyl) is preferred.

Examples of the "heteroatom" in the "hydrocarbon residue which may link via a heteroatom and may be substituted" represented by R³ include -O-, -S(O)ₘ- [m represents an integer of 0 to 2], -NR'- [R' represents hydrogen atom or lower(C₁₋₄)alkyl] and the like, and among these, -O- is preferably used.

Among the above-mentioned groups, as R³, preferred are lower(C₁₋₅)alkyl or lower(C₂₋₅)alkenyl group and the like, each of which may be linked via -O-, -S(O)ₘ- [m represents an integer of 0 to 2] or -NR'- [R' represents hydrogen atom or lower(C₁₋₄)alkyl] and may be substituted with substituents selected from hydroxy group, amino group, halogen and lower(C₁₋₄)alkoxy group. Among these, lower(C₁₋ ₅)alkyl or lower(C₁₋₅)alkoxy (more preferably ethoxy) is preferred.

Among the compounds having angiotensin II antagonistic activity represented by the formula (I), preferred is a benzimidazole-7-carboxylic acid derivative represented by the formula (I'): 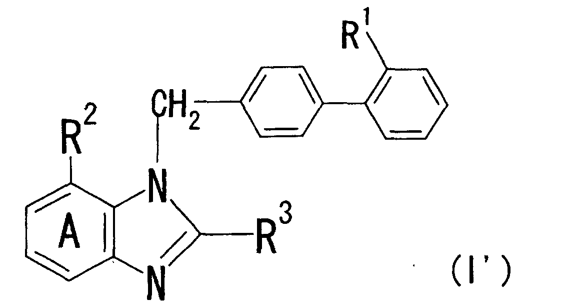 wherein R¹ is (1) carboxyl group, (2) tetrazolyl group or (3) a group represented by the formula: 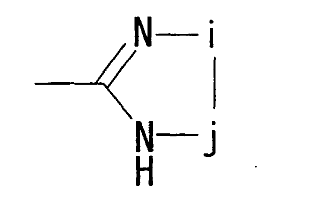 wherein i represents -O- or -S-, j represents >=O, >=S or >=S(O)ₘ, m is as defined above], ring A represents a benzene ring optionally substituted with optionally substituted lower(C₁₋₄)alkyl (e.g., lower (C₁₋₄)alkyl optionally substituted with hydroxy group, carboxyl group, halogen and the like) or halogen and the like, in addition to the substituent R² (preferably a benzene ring that does not have any substituent except for R²), R² represents a group represented by the formula: -CO-D [wherein D represents (1) hydroxy group or (2) lower(C₁₋₄)alkoxy wherein the alkyl portion is optionally substituted with hydroxy group, amino, halogen, lower(C₂₋₆)alkanoyloxy (e.g., acetoxy, pivaloyloxy and the like), lower(C₃₋ ₈)cycloalkanoyloxy, lower(C₁₋₆)alkoxycarbonyloxy (e.g., methoxycarbonyloxy, ethoxycarbonyloxy and the like), lower (C₃₋₈) cycloalkoxycarbonyloxy (e.g., cyclohexyloxycarbonyloxy and the like), lower(C₁₋₄)alkoxy or lower(C₃₋₈)cycloalkoxy], R³ is lower(C₁₋₅)alkyl or lower(C₂₋ ₅)alkenyl group which may link via -O-, -S(O)ₘ- [m represents an integer of 0 to 2] or -NR'- [R' represents hydrogen atom or lower(C₁₋₄)alkyl] and may be substituted with substituent(s) selected from hydroxy group, amino group, halogen and lower(C₁₋₄)alkoxy group (preferably lower(C₁₋₅)alkyl or lower(C₁₋₅)alkoxy; more preferably ethoxy)], or a pharmacologically acceptable salt thereof. Among these, 2-ethoxy-1-[[2'-(1H-tetrazol-5-yl)biphenyl-4-yl]methyl]benzimidazole-7-carboxylic acid [Candesartan], 1-(cyclohexyloxycarbonyloxy)ethyl 2-ethoxy-1-[[2'-(1H-tetrazol-5-yl)biphenyl-4-yl]methyl]benzimidazole-7-carboxylate [Candesartan cilexetil], pivaloyloxymethyl 2-ethoxy-1-[[2'-(1H-tetrazol-5-yl)biphenyl-4-yl]methyl]benzimidazole-7-carboxylate, 2-ethoxy-1-[[2'-(2,5-dihydro-5-oxo-1,2,4-oxadiazol-3-yl)biphenyl-4-yl]methyl]benzimidazole-7-carboxylic acid or a salt thereof and the like are preferred.

The above-mentioned benzimidazole derivative can be synthesized by known methods, such as methods disclosed in EP-425921, EP-459136, EP-553879, EP-578125, EP-520423, EP-668272 and the like, or a similar method thereto, and the like. When Candesartan cilexetil is used, it is preferable to use the stable C type crystals disclosed in EP-459136.

The "compound having angiotensin II antagonistic activity" as used in the present invention may be a compound that inhibits the binding of angiotensin II to its receptor on a cell membrane competitively or non-competitively resulting in attenuation of strong blood vessel contraction and blood vessel smooth muscle proliferation induced by angiotensin II, thereby alleviating conditions of hypertension, and the like.

While the "compound having angiotensin II antagonistic activity" may be peptidic or nonpeptidic, a nonpeptidic compound having antagonistic activity is preferred, since it has an advantage of longer action period. Examples of such a nonpeptidic compound having antagonistic activity include the above-mentioned compound represented by the formula (I). Furthermore, as the compound having angiotensin II antagonistic activity, a compound having an oxygen atom in the molecule is preferred. Among these, a compound having an ether bond or a carbonyl group (said carbonyl group may form a hydroxy group by resonance) is more preferred, a compound having an ether bond or a ketone derivative is still more preferred, and an ether derivative is especially preferred.

The nonpeptidic compound having angiotensin II antagonistic activity is not specifically limited in so far as the object of the present invention is achieved. Imidazole derivatives are disclosed in JP 56-71073 A, JP 56-71074 A, JP 57-98270 A, JP 58-157768 A, USP 4,355,040, USP 4,340,598 and the like; modified imidazole derivatives are disclosed in EP-253310, EP-291969, EP-324377, EP-403158, WO-9100277, JP 63-23868 A, JP 1-117876 A and the like; pyrrole, pyrazole and triazole derivatives are disclosed in USP 5,183,899, EP-323841, EP-409332, JP 1-28707 A, and the like; benzimidazole derivatives are disclosed in USP 4,880,804, EP-0392317, EP-0399732, EP-0400835, EP-425921, EP-459136, JP 3-63264 A, and the like; azaindene derivatives are disclosed in EP-399731, and the like; pyrimidone derivatives disclosed in EP-407342, and the like; quinazoline derivatives are disclosed in EP-411766, and the like; xanthine derivatives are disclosed in EP-430300, and the like; fused imidazole derivatives are disclosed in EP-434038, and the like; pyrimidinedione derivatives are disclosed in EP-442473, and the like, thienopyridone derivatives are disclosed in EP-443568, and the like; furthermore, heterocyclic compounds are disclosed in EP-445811, EP-483683, EP-518033, EP-520423, EP-588299, EP-603712, and the like. The representative compounds of these are also disclosed in Journal of Medicinal Chemistry, vol. 39, 3, pp.625-656, 1996. When the nonpeptidic compound having angiotensin II antagonistic activity is used, any nonpeptidic compound may be used in so far as it has angiotensin II antagonistic activity, in addition to the compounds disclosed in the above-mentioned known documents. Among these, Losartan (DuP753), Potassium Losartan, Eprosartan (SK&F108566), Candesartan cilexetil (TCV-116), Valsartan (CGP-48933), Telmisartan (BIBR277), Irbesartan (SR47436), Olmesartan (CS-866) also known as Olmesartan medoxomil, Olmesartan (including RNH-6270), Tasosartan (ANA-756) and metabolic activated substances thereof (Candesartan and the like) are preferably used.

The compound having angiotensin II antagonistic activity to be used in the present invention, such as the benzimidazole derivative represented by the formula (I) and the like, or a prodrug thereof may be as it is, or may be in the form of a pharmacologically acceptable salt thereof. Examples of such a salt include, when the compound having angiotensin II antagonistic activity has an acidic group such as carboxyl group and the like, a salt with an inorganic base (e.g., alkaline metal such as sodium, potassium, etc., alkaline earth metal such as calcium, magnesium, etc., transition metal such as zinc, iron, copper, etc., and the like) and a salt with an organic base (e.g., organic amine such as trimethylamine, triethylamine, pyridine, picoline, ethanolamine, diethanolamine, triethanolamine, dicyclohexylamine, N,N'-dibenzylethylenediamine, etc., basic amino acid such as alginine, lysine, ornithine, etc., and the like), and the like.

When the compound having angiotensin II antagonistic activity has a basic group such as amino group, examples of the salt include a salt with an inorganic acid or organic acid (e.g., hydrochloric acid, nitric acid, sulfuric acid, phosphoric acid, carbonic acid, hydrogencarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.), a salt with acidic amino acid such as asparatic acid, glutamic acid, etc., and the like.

Specifically, for example, it is particularly preferred that the compound having angiotensin II antagonistic activity is Losartan, Potassium Losartan and the like.

A prodrug of the compound having angiotensin II antagonistic activity used in the present invention [hereinafter sometimes abbreviated as All antagonist compound] refers to a compound that is converted into the All antagonist compound by a reaction with an enzyme, gastric acid, or the like under physiological conditions in the living body, namely, a compound that is converted into the All antagonist compound by an enzymatic oxidation, reduction, hydrolysis, or the like, and a compound that is converted into the All antagonist compound by hydrolysis with gastric acid or the like. Examples of the prodrug of the All antagonist compound include the All antagonist compound whose amino group is acylated, alkylated, or phosphorylated (e.g., the All antagonist compound whose amino group is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, tert-butylated and the like); the All antagonist compound whose hydroxy group is acylated, alkylated, phosphorylated, or borated (e.g., the All antagonist compound whose hydroxy group is acetylated, palmitoylated, propanoylated, pivaloylated, succinylated, fumarylated, alanylated, dimethylaminomethylcarbonylated and the like); the All antagonist compound whose carboxyl group is esterified or amidated (e.g., the All antagonist compound whose carboxyl group is ethylesterified, phenylesterified, carboxymethylesterified, dimethylaminomethylesterified, pivaloyloxymethylesterified, ethoxycarbonyloxyethylesterified, phthalidylesterified, (5-methyl-2-oxo-1,3-dioxolen-4-yl)methylesterified, cyclohexyloxycarbonyloxyethylesterified, methylamidated, and the like); and the like. These compounds can be prepared from the All antagonist compounds by a per se known method.

Further, a prodrug of the All antagonist compound may be a compound that is converted into the All antagonist compound under the physiological conditions as described in "Iyakuhin no Kaihatu (Development of Drugs)", Vol. 7, Bunshi Sekkei (Molecular Design), Hirokawa Shoten, 1990, pages 163-198.

The All antagonist compound may be a hydrate or anhydrate.

The compound having angiotensin II antagonistic activity to be used in the present invention such as the compound represented by the formula (I) and the like, and a pharmacologically acceptable salt thereof, have low toxicity, and they can be used as an agent for preventing or treating portal hypertension for mammals (e.g., human, mouse, rat, rabbit, dog, cat, cattle, swine, simian and the like) directly or by mixing with a pharmacologically acceptable carrier to obtain a pharmaceutical composition.

Furthermore, hepatic fibrosis can be suppressed by administering the agent for preventing or treating portal hypertension to the above-mentioned mammals.

Here, various organic or inorganic carrier substances that have been conventionally used as raw materials for pharmaceutical preparations are used as the pharmacologically acceptable carrier. They are incorporated into a composition as excipient, lubricant, binder, disintegrating agent for a solid preparation; solvent, dissolution aid, suspending agent, isotonic agent, buffer, soothing agent for a liquid preparation, and the like. If necessary, additives for preparations such as antiseptic agent, antioxidant, coloring agent, sweetening agent and the like can also be used.

Preferred examples of the excipient include lactose, sucrose, D-mannitol, D-sorbitol, starch, α starch, dextrin, crystalline cellulose, hydroxypropyl cellulose, carboxymethyl cellulose sodium, gum arabic, dextrin, Pullulan, light anhydrous silicic acid, synthetic aluminum silicate, magnesium aluminomethasilicate and the like.

Preferred examples of the lubricant include magnesium stearate, calcium stearate, talc, colloidal silica and the like.

Preferred examples of the binder include α starch, sucrose, gelatin, gum arabic, methylcellulose, carboxymethylcellulose, carboxymethylcellulose sodium, crystalling cellulose, sucrose, D-mannitol, trehalose, dextrin, Pullulan, hydroxypropyl cellulose, hydroxypropylmethyl cellulose, polyvinyl pyrrolidone and the like.

Preferred examples of the disintegrating agent include lactose, sucrose, starch, carboxymethylcellulose, carboxymethylcellulose calcium, sodium crosscarmellose, carboxymethylstarch sodium, light anhydrous silicic acid, hydroxypropyl cellulose and the like.

Preferred examples of the solvent include water for injection, saline, Ringer's injection, alcohol, propylene glycol, polyethylene glycol, sesame oil, corn oil, olive oil, cottonseed oil and the like.

Preferred examples of the dissolution aid include polyethylene glycol, propylene glycol, D-mannitol, trehalose, benzyl benzoate, ethanol, trisaminomethane, cholesterol, triethanol amine, sodium carbonate, sodium citrate, sodium salicylate, sodium acetate and the like.

Preferred examples of the suspending agent include surfactants such as stearyltriethanol amine, sodium lauryl sulfonate, lauryl aminopropionate, lecithin, benzalkonium chloride, benzethonium chloride, glycerine monostealate and the like; hydrophilic polymers such as polyvinyl alcohol, polyvinyl pyrrolidone, carboxymethylcellulose sodium, methylcellulose, hydroxymethylcellulose, hydroxyethylcellulose, hydroxypropylcellulose and the like; polysolvates, polyoxyethylenehydrogenated castor oil and the like.

Preferred examples of the isotonicity agent include sodium chloride, glycerine, D-mannitol, D-sorbitol, glucose and the like.

Preferred examples of the buffer include phosphate buffer, acetate buffer, carbonate buffer, citrate buffer and the like.

Preferred examples of the soothing agent include benzylalcohol and the like.

Preferred examples of the antiseptic agent include paraoxy benzoates, chlorobutanol, benzylalcohol, phenethylalcohol, dehydroacetic acid, sorbic acid and the like.

Preferred examples of the antioxidant include sulfite, ascorbate and the like.

Preferred examples of the coloring agent include water-soluble edible tar pigments (e.g., Food Color Red Nos. 2 and 3, Food Color Yellow Nos. 4 and 5, Food Color Blue Nos. 1 and 2 and the like, water insoluble lake pigments (e.g., aluminum salt of the above-mentioned water-soluble edible tar pigment and the like), natural pigments (e.g., β-carotene, chlorophyll, iron oxide red and the like) and the like.

Preferred examples of the sweetening agent include saccharin sodium, dipotassium glycyrrhizinate, aspartame, stevia and the like.

Examples of the dosage form of the aforementioned pharmaceutical composition include agents for oral administration such as tablets, capsules (including soft capsules and microcapsules), granules, powders, syrups, emulsions, suspensions and the like; and agents for parenteral administration such as injectable preparations (e.g., subcutaneous injections, intravenous injections, intramuscular injections, intraperitoneal injections, intravitreous and the like), drops, external agents (e.g., transnasal preparations, transdermal preparations, ointments and the like), suppositories (e.g., rectal suppositories, vaginal suppositories and the like), pellets, drops, sustained-release preparations and the like. These may be administered safely via oral or parenteral administration route.

The pharmaceutical composition can be produced according to a method conventionally used in the field of pharmaceutical preparations, such as the method described in Japan Pharmacopoeia and the like. Hereinafter, a specific method for preparing the pharmaceutical preparation will be described in detail.

The content of the compound of the formula (I) or a pharmacologically acceptable salt thereof in the pharmaceutical composition is about 0.001% by weight to about 95% by weight, preferably about 0.1% by weight to 70% by weight based on the total amount of the composition.

For example, an agent for oral administration is prepared by adding, to the active component, excipient (e.g., lactose, sucrose, starch, D-mannitol and the like), disintegrant (e.g., calcium carboxymethylcellulose and the like), binder (e.g., α starch, gum arabic, carboxymethylcellulose, hydroxypropylcellulose, polyvinylpyrrolidone and the like), lubricant (e.g., talc, magnesium stearate, polyethylene glycol 6000 and the like) and the like, compression-molding the resultant mixture, and, if necessary, coating the same using a coating base for masking of taste, enteric property or sustained release according to a per se known method.

Examples of the coating base include a dragee base, a water-soluble film coating base, an enteric film coating base, a sustained release film coating base and the like.

As the dragee base, sucrose may be used, optionally, together with one or two materials selected from talc, precipitated calcium carbonate, gelatin, gum arabic, pullulan, carnauba wax and the like.

Examples of the water-soluble film coating base include cellulose polymers such as hydroxypropylcellulose, hydroxypropylmethylcellulose, hydroxyethylcellulose, methylhydroxyethylcellulose and the like; synthetic polymers such as polyvinyl acetal diethylaminoacetate, aminoalkyl methacrylate copolymer E [Eudragit E, tradename, Rohm Pharma], polyvinylpyrrolidone and the like; polysaccharides such as pullulan and the like; and the like.

Examples of the enteric film coating base include cellulose polymers such as hydroxypropylmethylcellulose phthalate, hydroxypropylmethylcellulose acetate succinate, carboxymethylethylcellulose, acetic phthalic cellulose and the like; acrylic acid polymers such as methacrylic acid copolymer L [Eudragit L, tradename, Rohm Pharma], methacrylic acid copolymer LD [Eudragit L-30D55, tradename, Rohm Pharma], methacrylic acid copolymer S [Eudragit S, tradename, Rohm Pharma] and the like; naturally occurring substances such as shellac and the like; and the like.

Examples of the sustained release film coating base include cellulose polymers such as ethylcellulose and the like; acrylic acid polymers such as aminoalkyl methacrylate copolymer RS [Eudragit RS, tradename, Rohm Pharma], ethyl acrylate·methyl methacrylate copolymer suspension [Eudragit NE, tradename, Rohm Pharma] and the like, and the like.

Two or more kinds of the above-mentioned coating bases may be mixed in an appropriate ratio for use. In addition, a light screen such as titanium oxide, iron tri or dioxide and the like may be used upon coating.

An injectable preparation is prepared by dissolving, suspending or emulsifying an active component in an aqueous solvent (e.g., distilled water, physiological saline, Ringer's solution and the like) or an oily solvent (e.g., plant oil such as olive oil, sesame oil, cottonseed oil, corn oil and the like, propylene glycol and the like) and the like, together with a dispersing agent (e.g., polysorbate 80, polyoxyethylene hydrogenated castor oil 60 and the like), polyethylene glycol, carboxymethylcellulose, sodium alginate and the like), preservative (e.g., methylparaben, propylparaben, benzyl alcohol, chlorobutanol, phenol and the like), isotonicity agent (e.g., sodium chloride, glycerol, D-mannitol, D-sorbitol, glucose and the like) and the like. At this time, if desired, a dissolution aid (e.g., sodium salicylate, sodium acetate and the like), a stabilizer (e.g., human serum albumin and the like), a soothing agent (e.g., benzyl alcohol and the like) and the like may be used.

While a dose of the compound of the formula (I) of the present invention and a pharmacologically acceptable salt thereof varies depending on particular administration subject, administration route, disease condition and the like, the compound of the formula (I) of the present invention or a pharmacologically acceptable salt thereof or a prodrug thereof as an active component is generally given in a single dose of about 0.001 to 500 mg, preferably 0.1 to 100 mg, in the case of, for example, oral administration to a mammal, especially an adult (50 kg body weight). This dose is desirably given 1 to 3 times a day.

The above-mentioned "compound having angiotensin II antagonistic activity", preferably a compound represented by the formula (I) or a salt thereof or a prodrug thereof, may be applied as a safe sustained release preparation for preventing or treating portal hypertension with a biodegradable polymer. Such a sustained release preparation can be prepared according to a per se known method. For example, it may be prepared according to the methods disclosed in WO99/44590, WO01/60410, Japanese Patent Application No. 11-351798 and the like and applied for preventing or treating portal hypertension.

Examples of such a sustained release preparation include:
[1] a sustained release preparation comprising a compound having angiotensin II antagonistic activity (e.g., a compound represented by the formula (I)) or a salt thereof and a biodegradable polymer;
[2] the sustained release preparation according to the above [1], wherein the biodegradable polymer is an α-hydroxycarboxylic acid polymer;
[3] the sustained release preparation according to the above [1], wherein the α-hydroxycarboxylic acid polymer is lactic acid-glycolic acid polymer;
[4] the sustained release preparation according to the above [3], wherein the composition molar ratio of lactic acid and glycolic acid is 100/0 to 40/60;
[5] the sustained release preparation according to the above [2], wherein a weight average molecular weight of the polymer is 3,000 to 50,000;
[6] the sustained release preparation according to the above [1], which is for injection;
[7] the sustained release preparation according to the above [1] which contains a polyvalent metal;
[8] the sustained release preparation according to the above [7], wherein the polyvalent metal is zinc, or
[9] A sustained release preparation comprising a compound having angiotensin II antagonistic activity (e.g., a compound represented by the formula (I)) or a salt thereof, a biodegradable polymer and a polyvalent metal.

Such a sustained release preparation is produced and used according to the method disclosed in WO99/44590.

Other aspects of the sustained release preparation include:
[1] a sustained release preparation comprising a compound having angiotensin II antagonistic activity (e.g., a compound represented by the formula (I)) or a salt thereof, a component that have been obtained by treating a water insoluble polyvalent metal compound with water, and a biodegradable polymer;
[2] the sustained release preparation according to the above [1], wherein the biodegradable polymer is α-hydroxycarboxylic acid polymer;
[3] the sustained release preparation according to the above [2], wherein the α-hydroxycarboxylic acid polymer is lactic acid-glycolic acid polymer;
[4] the sustained release preparation according to the above [3], wherein the composition molar ratio of lactic acid and glycolic acid is 100/0 to 40/60;
[5] the sustained release preparation according to the above [2], wherein the weight average molecular weight of the polymer is 3,000 to 50,000;
[6] the sustained release preparation according to the above [1], which is for injection;
[7] the sustained release preparation according to the above [1], wherein the polyvalent metal is zinc,
[8] the sustained release preparation according to the above [1], wherein the polyvalent metal compound is zinc oxide;
[9] the sustained release preparation according to the above [1] which further comprises a polyvalent metal; or
[10] the sustained release preparation according to the above [9], wherein the polyvalent metal is zinc.

Such a sustained release preparation is prepared and used according to the method disclosed in WO 01/60410.

The sustained release preparation of the present invention can be administrated directly, or by incorporating the preparation as a raw material into various dosage forms, such as an injectable or implantable preparation for intramuscular, subcutaneous, organ and the like, transmucosal preparation for nasal cavity, rectal, uterus and the like, an oral preparation such as a solid preparation (e.g., capsule (e.g., hard capsule, soft capsule and the like), granules, powder and the like, a liquid preparation such as syrup, emulsion, suspension and the like), and the like. Furthermore, the preparation can be administrated by a jet injector.

For example, in order to formulate the sustained release preparation of the present invention in the form of an injectable preparation, it can be formulated into an aqueous suspension together with a dispersing agent (e.g., surfactant such as Tween 80, HCO-60 and the like, polysaccarides such as sodium hyarulonate, carboxymethyl cellulose, sodium alginate and the like), a preservative (e.g., methylparaben, propylparaben and the like), an isotonic agents (e.g., sodium chloride, mannitol, sorbitol, glucose, proline and the like) and the like, or can be formulated into an oily suspension by dispersing it with a vegetable oil such as sesame oil, corn oil and the like, to obtain a sustained release injectable preparation that can be practically used.

The particle size of the sustained release preparation of the present invention may be in such a range that the dispersibility and needle penetration property are satisfied, when it is used as a suspended injectable preparation. For example, the mean particle diameter is in the range of about 0.1 to 300 µm, preferably in the range of about 0.5 to 150 µm, more preferably in the range of about 1 to 100 µm.

Examples of a method for formulating the sustained release preparation of the present invention as an aseptic preparation include, but not limited to, a method for carrying out all the production steps aseptically, gamma ray sterilization, addition of an antiseptic agent and the like.

Since the sustained release preparation of the present invention has low toxicity, it can be used as safe medicament and the like for mammals (e.g., human, cattle, swine, dog, cat, mouse, rat, rabbit and the like).

While a dose of the sustained release preparation of the present invention varies depending on particular kind, content, dosage form of the compound having All antagonistic activity as the base component, duration period of the compound having All antagonistic activity, disease condition, subject animal and the like, the dose may be such that an effective amount of the compound having All antagonistic activity is lasting. The dose of the compound having All antagonistic activity as the base component per single dose may be suitably selected from, for example, in case that the sustained release preparation is a one-month preparation, preferably in the range of about 0.01 mg to 50 mg/kg body weight, more preferably in the range of about 0.1 mg to 30 mg/kg body weight per an adult.

The frequency of administration can be suitably selected from once per several weeks, once per one month or once per several months (e.g., three months, four months, six months and the like) and the like, depending on particular kind, content, dosage form of the compound having All antagonistic activity as the base component, duration period of release of the compound having All antagonistic activity, disease condition, subject animal and the like.

The sustained release preparation of the present invention can advantageously used as an agent for preventing or treating portal hypertension and can maintain a constant blood level in both day and night. Then, the dose and the frequency of administration can be reduced in comparison with the administration of an oral agent. Furthermore, stable decrease in portal vein pressure can be expected because of the less change in blood level of the drug. It has been known that rhexis of esophageal varices is of frequent occurrence in night (Hepatology 1994; 19: 595-601), and the preparation of the present invention therefore is an advantageous prophylactic agent of esophageal and gastric varices rhexis. This is a characteristic of the preparation. Moreover, since the change in a symptom due to the intermittence of taking the drug and the like hardly occurs, a distinct therapeutic can also be expected.

The agent for preventing or treating portal hypertension of the present invention may be used in combination with a drug for the treatment of hepatic diseases. In this case, examples of possible components for combination include glycyrrhizin preparations [e.g., Stronger Neo-Minophagen and the like], lever hydrolysates, SH compounds [e.g., glutathione and the like], special amino acid preparations [e.g., Aminoleban and the like], phospholipids [e.g., polyene phosphatidyl choline and the like], vitamins [e.g., vitamin B₁, B₂, B₆, B₁₂, C and the like], adenocorticotropic hormones [e.g., dexamethasone, betamethasone and the like], interferons [e.g., interferon α, β and the like], drugs for treating hepatocerebral encephalopathy [e.g., lactulose and the like], hemostatics used during rhexis of esophageal or gastric varices [e.g., vasopressin, somatostatin and the like].

Furthermore, it may be used in combination with other medicament components such as vasodilators, therapeutic agents for hyperlipidemia, therapeutic agent for hypertension, therapeutic agents for chronic cardiac failure, therapeutic agents for nephrotic syndrome, therapeutic agents for chronic renal failure, therapeutic agents for gastric and duodenal ulcer, therapeutic agents for biliary tract disease, antitumor agent, therapeutic agents for infectious disease, therapeutic agents for thrombogenesis or anti-inflammatory agent. In this case, these compounds can be administered as an oral preparation and, if desired, they can be administered in the form of suppository as a rectal preparation. In this case, examples of the possible component for combination include β receptor blockers [e.g., propranolol, nipradilol, atenolol, carvedilol and the like], α receptor modifiers [e.g., prazosin, clonidine and the like], nitrous acid drug [e.g., nitroglycerin, isosorbide dinitrate and the like], diuretic [e.g., spironolactone, furosemide, chlorothiazide and the like], endothelin antagonists.

Furthermore, the following therapeutic agents can be combined.

Vasodilators: nifedipine, diltiazem, nicorandil, nitrous acid drug and the like;

Therapeutic agents for hypertension: ACE inhibitors [e.g., maleic enalapril and the like], Ca antagonist [e.g., manidipine, amlodipine and the like] and the like; Therapeutic agents for hyperlipidemia: HMG-CoA reductase inhibitors [e.g., atorovastatin, cerivastatin and the like], fibrate drugs [e.g., clofibrate, bezafibrate and the like], squalene synthase inhibitors and the like;

Therapeutic agents for chronic cardiac failure: cardiac restoratives [e.g., cardiotonic glycoside (digoxin and the like), PDE inhibitor and the like], ACE inhibitors [e.g., maleic enalapril and the like], Ca antagonists [e.g., amlodipine and the like] and β receptor blocker and the like;

Therapeutic agents for chronic renal failure: antihypertensive drugs [e.g., ACE inhibitor (maleic enalapril and the like) and Ca antagonist (manidipine and the like), α receptor blocker and the like] and the like;

Therapeutic agents for gastric and duodenal ulcer: antacid [e.g., histamine H₂ antagonist (cimetidine and the like), proton pump inhibitors (lansoprazole and the like) and the like];

Therapeutic agents for biliary tract disease: choleretics [e.g., dehydrocholic acid and the like], cholekinetics [e.g., magnesium sulfate and the like] and the like;

Antitumor drugs: alkylating agent, antimetabolite, antitumor antibiotic preparation, antitumor plant component preparation and the other antitumor drugs and the like;

Therapeutic agents for infectious diseases: [e.g., antibiotic preparations (cefotiam hydrochloride, cefozopran hydrochloride, ampicillin and the like), chemotherapeutics (sulfa drug, synthetic antibacterial agent, antiviral agents and the like), biological preparations (vaccines, blood preparations such as immunoglobulin and the like) and the like] and the like;

Therapeutic agents for thrombogenesis: anticoagulant drugs [e.g., heparin sodium, heparin calcium, warfarin calcium, drugs having a function for correcting the balance of blood coagulation factor Xa inhibitor and clotted fibrinolytic system, and the like], thrombolytic agents [e.g., tPA, urokinase], anti-platelet agents [e.g., aspirin, sulfinpyrazolo, dipyridamol, ticlopidine (panaldine), cilostazol, GPIIb/IIIa antagonist and the like] and the like;

Antiinflammatory agents: aspirin, acetoaminophene, non-steroid antiinflammately agents [e.g., indomethacin and the like], steroid agents [e.g., dexamethasone and the like] and the like;

The agent of the present invention may be used together with these drugs simultaneously or separately at intervals.

When these drugs are used in combination, each of the drugs can be orally or parenterally administrated as pharmaceutical composition(s), individually or simultaneously, by mixing them with a pharmacologically acceptable carrier, excipient, binder, dilution agent and the like to formulate them into preparation(s). When the drugs are individually formulated, the individually formulated drugs can be administrated by mixing with a dilution agent and the like upon use, or the individually formulated preparations can be administrated to the same subject simultaneously or separately at intervals. The medicament of the present invention also include a kit product for mixing the individually formulated drugs with a dilution agent and the like upon use and administrating them to the same subject (for example, a kit for injection comprising ampoules containing individual powdery drugs and a dilution agent for mixing and dissolving the two or more of drugs upon use, and the like), a kit product for administering the individually formulated preparations to the same object simultaneously or separately at intervals (for example, a kit for administrating two or more of tablets simultaneously or separately at intervals, in which the tablets each containing individual drug are put into the same bag or separate bags, and optionally having a label on which the period for administration of drugs has been provided, etc.) and the like.

Hereinafter the present invention will be more specifically explained with referring to Examples and Experimental Examples, but they do not limit the scope of the present invention.

An agent for preventing or treating portal hypertension comprising the compound having All antagonistic activity according to the present invention as an effective component can be prepared by the following formulation.

### Example 1

2-Ethoxy-1-[[2'-(4,5-dihydro-5-oxo-1,2,4-oxadiazol-3-yl)biphenyl-4-yl]methyl]benzimidazole-7-carboxylic acid (hereinafter abbreviated as compound A) (0.25 g) and lactic acid-glycolic acid copolymer (lactic acid/glycolic acid=75/25 (mol%), weight-average molecular weight of 10,700, number-average molecular weight of 6,100, number-average molecular weight by end group quantification of 3,770, manufactured by Wako Pure Chemical Co., Ltd.) (2.25 g) were dissolved in a mixed solvent of dichloromethane (3.5 ml) and methanol (1.5 ml), and the mixture was poured into 0.1% (w/w) aqueous solution of polyvinyl alcohol (500 ml) that had been previously adjusted to 18°C. Using a turbine type homomixer, an O/W emulsion was prepared at 7,000 rpm. This O/W emulsion was stirred at room temperature for 3 hours to vaporize dichloromethane and methanol, and the oil phase was solidified and collected using a centrifuge at 2,000 rpm. This was dispersed again in distilled water and further centrifuged, and the liberated drug and the like were washed out. To the collected microcapsules were added a small amount of distilled water and dispersed again, and the dispersant was lyophilized to obtain a powder. The ratio of collection was 69%, the percentage of encapsulation of compound A in the microcapsules was 92%, and the content of the compound A was 9.2%.

### Example 2

A solution of bisodium salt of compound A (0.25 g) in distilled water (0.4 ml) was mixed with a solution of lactic acid-glycolic acid copolymer (same as the Example 1) (2.25 g) in dichloromethane (4 ml), and the mixture was emulsified with a homogenizer to form a W/O emulsion. The W/O emulsion was then poured into 0.1% (w/w) aqueous solution of polyvinyl alcohol (500 ml) that had been previously adjusted to 18°C, and a W/O/W emulsion was prepared using a turbine type homomixer at 7,000 rpm. The W/O/W emulsion was stirred at room temperature for 3 hours to volatilize dichloromethane, and the oil phase was solidified and collected using a centrifuge at 2,000 rpm. This was dispersed again in distilled water and further centrifuged, and the liberated drug and the like were washed out. The collected microcapsules were dispersed again by adding a small amount of distilled water and lyophilized to give a powder. The ratio of collection was 50%, the percentage of encapsulation of compound A in the microcapsules was 37%, and the content of compound A was 3.7%.

### Example 3

Compound A (0.4 g) and lactic acid polymer ethyl ester (a biodegradable polymer in which the terminal carboxy groups of lactic acid polymer have been ethyl esterified, weight-average molecular weight of 10,200, number-average molecular weight of 5,680, manufactured by Wako Pure Chemical Co., Ltd.) (1.6 g) were dissolved in a solution of dichloromethane (3.5 ml) and methanol (2.5 ml), and 0.1% (w/w) aqueous solution of polyvinyl alcohol (800 ml) containing 5% of mannitol, which had been previously adjusted to 18°C, and an O/W emulsion was prepared using a turbine type homomixer at 7,000 rpm turbine type homomixer. This O/W emulsion was stirred at room temperature for 3 hr to volatilize dichloromethane and methanol, and the oil phase was solidified and collected using a centrifuge at 2,000 rpm. This was dispersed again in distilled water and further centrifuged, and the liberated drug and the like were washed out. The collected microcapsules were dispersed again by adding a small amount of distilled water and lyophilized to obtain a powder. The ratio of collection was 83%, the percentage of encapsulation of compound A in the microcapsules was 86%, and the content of compound A was 17.1%.

### Example 4

2-Ethoxy-1-[[2'-(1H-tetrazol-5-yl)biphenyl-4-yl]methyl]benzimidazole-7-carboxylic acid (hereinafter abbreviated as compound B) (0.6 g) and zinc oxide having a particle size of 0.02 µm (0.09 g) were added to a solution of lactic acid-glycolic acid copolymer (lactic acid/glycolic acid 75/25 (mol%), weight-average molecular weight of 14,000, number-average molecular weight of 4,200, number-average molecular weight by end group quantification of 4,090, manufactured by Wako Pure Chemical Co., Ltd.) (2.4 g) in dichloromethane (4.5 ml) and ethanol (1 ml), and the mixture was stirred with shaking at room temperature for 12 hours to obtain a slightly clouded solution. The solution was poured into 0.1 wt% aqueous solution of polyvinyl alcohol (400 ml) that had been previously adjusted to 15°C, and an O/W emulsion was prepared using a turbine type homomixer at 7,000 rpm turbine type homomixer. This O/W emulsion was stirred at room temperature for 3 hr to volatilize dichloromethane and ethanol, and the oil phase was solidified and collected using a centrifuge at 2,000 rpm. This was dispersed again in distilled water and further centrifuged, and the liberated drug and the like were washed out. The collected microcapsules were dispersed again by adding a small amount of distilled water in which mannitol had been dissolved, and lyophilized to obtain a powder. The percentage of encapsulation of compound B in the microcapsules was 97%, and the content of compound B was 18.8%.

### Example 5

Microcapsules were obtained according to a manner similar to that of Example 1 except that the amount of zinc oxide was changed to 0.057 g. The percentage of encapsulation of compound B in the microcapsules was 97%, and the content of compound B was 19.0%.

### Example 6

Microcapsules were obtained according to a manner similar to that of Example 1 except that the amounts of compound B, zinc oxide and lactic acid-glycolic acid copolymer were changed to 0.9g, 2.1 g, 0.12 g, respectively. The percentage of encapsulation of compound B in the microcapsules was 96%, and the content of compound B was 27.8%.

### Example 7

Microcapsules were obtained according to a manner similar to that of Example 3 except that the amount of zinc oxide was changed to 0.18 g. The percentage of encapsulation of compound B in the microcapsules was 92%, and the content of compound B was 26.2%.

### Example 8

Compound B (1.8 g) and zinc oxide having a particle size of 0.02 µm (0.3 g) were added to a solution of lactic acid-glycolic acid copolymer (lactic acid/glycolic acid 75/25 (mol%), weight-average molecular weight of 14,000, number-average molecular weight of 4,200, number-average molecular weight by end group quantification of 4,090, manufactured by Wako Pure Chemical Co., Ltd.) (4.2 g) in dichloromethane (9 ml) and ethanol (1.5 ml), and the mixture was stirred with shaking at room temperature for 12 hr to give a slightly clouded solution. The solution was poured into 0.1 wt% aqueous solution of polyvinyl alcohol (800 ml) that had been previously adjusted to 15°C, and an O/W emulsion was prepared using a turbine type homomixer at 7,000 rpm turbine type homomixer. This O/W emulsion was stirred at room temperature for 3 hours to volatilize dichloromethane and ethanol, and the oil phase was solidified and collected using a centrifuge at 2,000 rpm. This was dispersed again in distilled water and further centrifuged, and the liberated drug and the like were washed out. The collected microcapsules were dispersed again by adding distilled water in which a small amount of mannitol had been dissolved, and lyophilized to give a powder. The percentage of encapsulation of compound B in the microcapsules was 94%, and the content of compound B was 26.8%.

### Example 9

Compound A (0.3 g) and zinc oxide having a particle size of 0.02 µm (0.05 g) were added to a solution of lactic acid-glycolic acid copolymer (lactic acid/glycolic acid 75/25 (mol%), weight-average molecular weight of 14,000, number-average molecular weight of 4,200, number-average molecular weight by end group quantification of 4,090, manufactured by Wako Pure Chemical Co., Ltd.) (0.7 g) in dichloromethane (1.5 ml) and methanol (1 ml), and the mixture wad stirred with shaking at room temperature for 12 hr to give a slightly clouded solution. The solution was poured into 0.1 wt% aqueous solution of polyvinyl alcohol (300 ml) that had been previously adjusted to 15°C, and an O/W emulsion was prepared using a turbine type homomixer at 2,000 rpm turbine type homomixer. This O/W emulsion was stirred at room temperature for 3 hours to volatilize dichloromethane and ethanol, and the oil phase was solidified and collected using a centrifuge at 2,000 rpm. This was dispersed again in distilled water and further centrifuged, and the liberated drug and the like were washed out. The collected microcapsules were dispersed again by adding distilled water in which a small amount of mannitol had been dissolved, and lyophilized to give a powder. The percentage of encapsulation of compound A in the microcapsules was 91%, and the content of compound A was 25.9%.

### Example 10

Compound B (1 g) and zinc oxide having a particle size of 0.02 µm (0.18 g) were added to a solution of lactic acid-glycolic acid copolymer (lactic acid/glycolic acid 75/25 (mol%), weight-average molecular weight of 14,000, number-average molecular weight of 4,200, number-average molecular weight by end group quantification of 4,090, manufactured by Wako Pure Chemical Co., Ltd.) (1.8 g) in dichloromethane (5 ml), and the mixture was emulsified with mixing using a small homogenizer for 60 seconds to give a slightly clouded dispersion. The dispersion was poured into 0.1 wt% aqueous solution of polyvinyl alcohol (400 ml) that had been previously adjusted to 15°C, and an O/W emulsion was prepared using a turbine type homomixer at 8,000 rpm. This O/W emulsion was stirred at room temperature for 3 hours to volatilize dichloromethane, and the oil phase was solidified and collected using a centrifuge at 2,000 rpm. This was dispersed again in distilled water and further centrifuged, and the liberated drug and the like were washed out. The collected microcapsules were dispersed again by adding a small amount of distilled water in which mannitol had been dissolved, and lyophilized to give a powder. The percentage of encapsulation of the compound B in the microcapsules was 96%, and the content of the compound B was 32.0%.

### Example 11

Microcapsules were obtained according to a manner similar to that of Example 7 except that a slightly clouded solution, which was obtained by adding ethanol (0.8 ml) to dichloromethane and shaken with shaking at room temperature for 12 hr, was used. The percentage of encapsulation of the compound B in the microcapsules was 95%, and the content of the compound B was 32.0%.

### Example 12

1-(Cyclohexyloxycarbonyloxy)ethyl 2-ethoxy-1-[[2'-(1H-tetrazol-5-yl)biphenyl-4-yl]methyl]benzimidazole-7-carboxylate (hereinafter abbreviated as compound C) (0.9 g) and lactic acid-glycolic acid copolymer (lactic acid/glycolic acid 75/25 (mol%), weight-average molecular weight of 14,000, number-average molecular weight of 4,200, number-average molecular weight by end group quantification of 4,090, manufactured by Wako Pure Chemical Co., Ltd.) (2.1 g) were dissolved in a mixed solvent of dichloromethane (4.5 ml) and ethanol (0.7 ml). To the solution was added zinc oxide having a particle size of 0.02 µm (0.15 g), and the mixture was stirred with shaking at room temperature for 12 hr to give a slightly clouded solution. This solution was poured into 0.1 wt% aqueous solution of polyvinyl alcohol (400 ml) that had been previously adjusted to 15°C, and an O/W emulsion was prepared using a turbine type homomixer at 7,500 rpm. This O/W emulsion was stirred at room temperature for 3 hours to volatilize dichloromethane and methanol, and the oil phase was solidified and collected using a centrifuge at 2,000 rpm. This was dispersed again in distilled water and further centrifuged, and the liberated drug and the like were washed out. The collected microcapsules were dispersed again by adding distilled water in which a small amount of mannitol had been dissolved, and lyophilized to give a powder. The percentage of encapsulation of the compound C in the microcapsules was 96%, and the content of the compound C was 27.4%.

### Example 13

Microcapsules were obtained according to a manner similar to that of Example 12 except that zinc oxide was not added. The percentage of encapsulation of compound C in the microcapsules was 98%, and the content of compound B was 30.0%.

### Example 14

Compound C (1.2 g) and lactic acid-glycolic acid copolymer (lactic acid/glycolic acid 75/25 (mol%), weight-average molecular weight of 14,000, number-average molecular weight of 4,200, number-average molecular weight by end group quantification of 4,090, manufactured by Wako Pure Chemical Co., Ltd.) (1.8 g) were dissolved in dichloromethane (5 ml). To the solution was added zinc oxide having a particle size of 0.02 µm (0.18 g), and the mixture was stirred with shaking at room temperature for 1 hr to give a slightly clouded solution. This solution was poured into 0.1 wt% aqueous solution of polyvinyl alcohol (400 ml) that had been previously adjusted to 15°C, and an O/W emulsion was prepared using a turbine type homomixer at 8,000 rpm. This O/W emulsion was stirred at room temperature for 3 hr to volatilize dichloromethane, and the oil phase was solidified and collected using a centrifuge at 2,000 rpm. This was dispersed again in distilled water and further centrifuged, and the liberated drug and the like were washed out. The collected microcapsules were dispersed again by adding a small amount of distilled water in which mannitol had been dissolved, and lyophilized to give a powder. The percentage of encapsulation of the compound C in the microcapsules was 95%, and the content of compound B was 35.9%.

### Example 15

Microcapsules were obtained according to a manner similar to that of Example 4 except that zinc oxide was not added. The percentage of encapsulation of compound B in the microcapsules was 99%, and the content of compound B was 19.8%.

### Example 16

Microcapsules were obtained according to a manner similar to that of Example 9 except that zinc oxide was not added. The percentage of encapsulation of compound B in the microcapsules was 95%, and the content of the compound B was 28.4%.

### Example 17

2-Ethoxy-1-[[2'-(lH-tetrazol-5-yl)biphenyl-4-yl]methyl]benzimidazole-7-carboxylic acid (compound B) (2 g) and zinc oxide (TYPE V, manufactured by Wako Pure Chemical Co., Ltd.) (0.36 g) were added to a solution of lactic acid-glycolic acid copolymer (lactic acid/glycolic acid 75/25 (mol%), weight-average molecular weight of 14,000, number-average molecular weight of 4,200, number-average molecular weight by end group quantification of 4,090, manufactured by Wako Pure Chemical Co., Ltd.) (3.6 g) in dichloromethane (11 ml) and ethanol (0.4 ml), and the mixture was stirred with shaking at room temperature for 14 hr to give a clouded solution. The solution was poured into 0.1 wt% aqueous solution of polyvinyl alcohol (800 ml) that had been previously adjusted to 15°C, and an O/W emulsion was prepared using a turbine type homomixer at 8.500 rpm using turbine type homomixer. This O/W emulsion was stirred at room temperature for 3 hr to volatilize dichloromethane and ethanol, and the oil phase was solidified and collected using a centrifuge at 2,000 rpm. This was dispersed again in distilled water and further centrifuged, and the liberated drug and the like were washed out. The percentage of encapsulation of compound B in the microcapsules was 98%, and the content of compound B was 33.0%.

### Example 18

Microcapsules were obtained according to a manner similar to that of Example 17 except that distilled water (0.4 ml) was added, and the stirring with shaking for 14 hr was changed to the dispersion (emulsifying) mixing with solids (compound B and zinc oxide) using a homogenizer at the same ppm for 1 min. The percentage of encapsulation of compound B in the microcapsules was 97%, and the content of compound B was 32.6%.

### Example 19

Microcapsules were obtained according to a manner similar to that of Example 18 except that the added amount of distilled water was changed to 0.08 ml. The percentage of encapsulation of compound B in the microcapsules was 97%, and the content of the compound B was 32.5%.

### Example 20

Compound B (4 g) and zinc oxide (TYPE V, manufactured by Wako Pure Chemical Co., Ltd.) (0.72 g) were added to a solution of lactic acid-glycolic acid copolymer (lactic acid/glycolic acid 75/25 (mol%), weight-average molecular weight of 10,600) (7.2 g) in dichloromethane (22 ml) and ethanol (0.8 ml), and distilled water (0.16 ml) was added thereto. Immediately after that, dispersion (emulsifying) mixing using a homogenizer was carried out in the condition similar to that of the Example 18 to give a clouded solution. The solution was developed onto a plane plate in a circle having a diameter of about 5 cm, and vacuum dried at room temperature for 15 hr to give a dried subject. The dried subject was roughly crushed on a sieve having a pore size of 250 µm. The sieved dried subject (5 g) and mannitol (0.4 g) was mixed, and the mixture was gas crushed using a jet mill apparatus (A-OJET, manufactured Seishin Enterprizes, Co., Ltd.) at the air pressure of 2 kg/cm² to give microparticles having a mean particle size of 21 µm. The content of the compound B in the particles was 31.0%.

### Example 21

A clouded solution obtained by dispersion (emulsifying) mixing according to the same formulation and steps as Example 20 was spray dried (Mobile Minor, manufactured by Niro Japan Co., Ltd.) in the following conditions to give microparticles having a mean particle size of 32 µm as a dried substance under cyclone.

| Method for spraying | two fluid nozzle (nozzle diameter 1.2 mm) |
|---|---|
| Air pressure | 1 kg/cm² |
| Temperature of inlet of the drying chamber | 90°C |
| Temperature of outlet of the drying chamber | 40-43°C |

The content of compound B in the microparticles was 28.1%.

### Example 22

| Capsule | |
|---|---|
| (1) Candesartan cilexetil | 30 mg |
| (2) Lactose | 90 mg |
| (3) Microcrystalline cellulose | 70 mg |
| (4) Magnesium stearate | 10 mg |
| One capsule | 200 mg |

| | |
|---|---|
| (1), (2) and (3) and 1/2 of (4) are mixed and granulated. To the mixture was added the residual (4) and the whole mixture is encapsulated in a gelatin capsule. | |

### Example 23

| Tablet | |
|---|---|
| (1) Candesartan cilexetil | 30 mg |
| (2) Lactose | 35 mg |
| (3) Corn starch | 150 mg |
| (4) Microcrystalline cellulose | 30 mg |
| (5) Magnesium stearate | 5 mg |
| One tablet | 250 mg |

| | |
|---|---|
| (1), (2), (3), 2/3 of (4) and 1/2 of (5) are mixed and granulated. To the granules are added the residual (4) and (5) and the mixture is compressed under pressure. | |

### Experimental Example 1

Effects for decreasing portal vein pressure in a rat portal hypertension model Method

Thioacetoamide (300 mg/kg, i.p.) is administered to male Wister rats (5 week-old) twice a week for 10 weeks to experimentally prepare hepatocirrhosis. Collection of blood with heparin is conducted from caudal vein, and the blood plasm GPT is measured. Based on the value, the rats are divided into two groups, and a vehicle or Candesartan sustained release preparation (for example, sustained-release preparations according to the above-mentioned Examples 1-21) is subcutaneously administrated (1.5 mg/rat, s.c.). After 4 weeks, each of the rats is anesthetized with pentobarbital, the abdomen is dissected and a catheter for measurement of pressure is indwelled in the portal vein. A catheter for measurement of blood pressure is indwelled in the femoral artery. After the pressure has been stabilized, the portal vein pressure and systemic blood pressure are measured, and the difference between the two groups is compared and discussed.

### INDUSTRIAL APPLICABILITY

The agent for preventing or treating portal hypertension of the present invention has excellent effects and does not show any side effects. Therefore, the agent has excellent properties as a medicament.

## Claims

1. An agent for preventing or treating portal hypertension which comprises a compound represented by the formula (I): 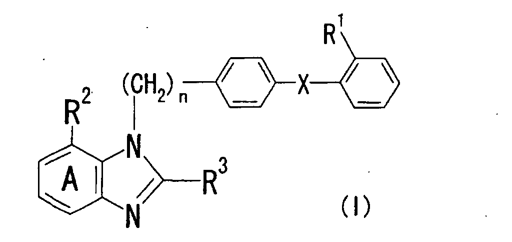 wherein R¹ represents a group capable of forming an anion or a group capable of converting to said group, X represents that the phenylene group and the phenyl group are linked directly or via a spacer having a chain of two or less of atoms, n represents 1 or 2, ring A represents a benzene ring which may be further substituted, R² represents a group capable of forming an anion or a group capable of converting to said group, R³ represents a hydrocarbon group which may link via a heteroatom and may be substituted, or a salt thereof, or a prodrug thereof.

2. An agent for preventing or treating portal hypertension comprising 2-ethoxy-1-[[2'-(1H-tetrazol-5-yl)biphenyl-4-yl]methyl]benzimidazole-7-carboxylic acid, or a salt thereof, or a prodrug thereof.

3. An agent for preventing or treating portal hypertension comprising 2-ethoxy-1-[[2'-(2,5-dihydro-5-oxo-1,2,4-oxadiazol-3-yl)biphenyl-4-yl]methyl]benzimidazole-7-carboxylic acid, or a salt thereof, or a prodrug thereof.

4. An agent for preventing or treating portal hypertension comprising 1-(cyclohexyloxycarbonyloxy)ethyl 2-ethoxy-1-[[2'-1H-tetrazol-5-yl)biphenyl-4-yl]methyl]benzimidazole-7-carboxylate or a salt thereof.

5. A sustained release agent for preventing or treating portal hypertension comprising a compound having All antagonistic activity, or a salt thereof, or a prodrug thereof.

6. The agent according to claim 5, wherein the compound having All antagonistic activity is a compound represented by the formula (I): 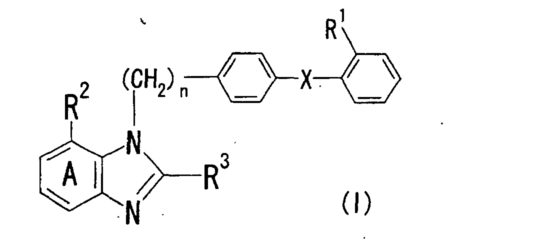 wherein R¹ represents a group capable of forming an anion or a group capable of converting to said group, X represents that the phenylene group and the phenyl group are linked directly or via a spacer having a chain of two or less of atoms, n represents 1 or 2, ring A represents a benzene ring which may be further substituted, R² represents a group capable of forming an anion or a group capable of converting to said group, R³ represents a hydrocarbon group which may link via a heteroatom and may be substituted.

7. The agent according to claim 5, wherein the compound having All antagonistic activity is 2-ethoxy-1-[[2'-(1H-tetrazol-5-yl)biphenyl-4-yl]methyl]benzimidazole-7-carboxylic acid.

8. The agent according to claim 5, wherein the compound having All antagonistic activity is 2-ethoxy-1-[[2'-(2,5-dihydro-5-oxo-1,2,4-oxadiazol-3-yl)biphenyl-4-yl]methyl]benzimidazole-7-carboxylic acid.

9. The agent according to claim 5, wherein the compound having AII antagonistic activity is 1-(cyclohexyloxycarbonyloxy)ethyl 2-ethoxy-1-[[2'-(1H-tetrazol-5-yl)biphenyl-4-yl]methyl]benzimidazole-7-carboxylate.

10. The agent according to claim 5, wherein the compound having All antagonistic activity or a salt thereof is Losartan, Potassium Losartan, Eprosartan, Candesartan cilexetil, Candesartan, Valsartan, Telmisartan, Irbesartan, Olmesartan or Tasosartan.

11. A method for preventing or treating portal hypertension which comprises administering an effective amount of a sustained release agent for preventing or treating portal hypertension comprising a compound having All antagonistic activity, or a salt thereof, or a prodrug thereof to a mammal.

12. Use of a compound having All antagonistic activity, or a salt thereof, or a prodrug thereof for manufacturing a sustained release agent for preventing or treating portal hypertension.

13. A method for suppressing hepatic fibrosis which comprises administering an effective amount of a sustained release agent comprising a compound having All antagonistic activity, or a salt thereof, or a prodrug thereof to a mammal.

14. A method for preventing or treating portal hypertension which comprises administering an agent for preventing or treating portal hypertension comprising a compound represented by the formula (I): 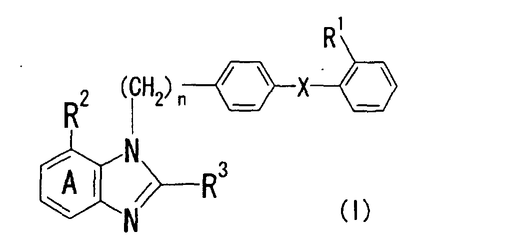 wherein R¹ represents a group capable of forming an anion or a group capable of converting to said group, X represents that the phenylene group and the phenyl group are linked directly or via a spacer having a chain of two or less of atoms, n represents 1 or 2, the ring A represents a benzene ring which may be further substituted, R² represents a group capable of forming an anion or a group capable of converting to said group, R³ represents a hydrocarbon group which may link via a heteroatom and may be substituted, or a salt thereof, or a prodrug thereof, to a mammal.

15. Use of a compound represented by the formula (I): 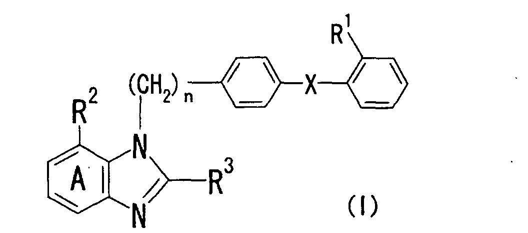 wherein R¹ represents a group capable of forming an anion or a group capable of converting to said group, X represents that the phenylene group and the phenyl group are linked directly or via a spacer having a chain of two or less of atoms, n represents 1 or 2, ring A represents a benzene ring which may be further substituted, R² represents a group capable of forming an anion or a group capable of converting to said group, R³ represents a hydrocarbon group which may link via a heteroatom and may be substituted, or a salt thereof, or a prodrug thereof, for manufacturing an agent for preventing or treating portal hypertension.
